# EUROPEAN PATENT SPECIFICATION

(11) **EP 3 362 428 B1**
(45) Date of publication and mention of the grant of the patent: **06.05.2020**
(21) Application number: 16856052.2
(22) Date of filing: 12.10.2016
(51) Int. Cl.: C07C 31/137, C07C 49/323, C07C 49/443, C07C 35/32, A61K 8/33, A61K 8/35, A61K 8/34, A61K 8/31, A61Q 13/00

(54) **NOVEL OCTAHYDROINDENYL PROPANAL COMPOUNDS**
NEUARTIGE OCTAHYDROINDENYLPROPANALVERBINDUNGEN
NOUVEAUX COMPOSÉS D'OCTAHYDRO-INDÈNYLE PROPANAL

(30) Priority: 12.10.2015 US 201562240238 P
(43) Date of publication of application: 22.08.2018
(73) Proprietor: International Flavors & Fragrances Inc., New York, NY 10019 (US)
(72) Inventor: JONES, Paul D., Aberdeen, New Jersey 07747 (US); BELKO, Robert P., Monroe, New Jersey 08831 (US); LEVORSE, JR., Anthony T., Westfield, New Jersey 07090 (US); GIFFIN, Nicole L., Hazlet, New Jersey 07730 (US); NARULA, Anubhav P.S., Hazlet, New Jersey 07730 (US)
(74) Representative: Barker Brettell LLP
(86) International application number: PCT/US2016/056486
(87) International publication number: WO 2017/066214

(56) References cited:
- EP-A2- 0 825 166
- EP-B1- 1 262 481
- US-A- 5 002 929
- US-A- 5 665 698
- US-A1- 2004 110 991
- US-B1- 6 271 193
- US-B1- 7 176 324

## Description

### Field of the Invention

The present invention relates to novel chemical entities and a method of using the same as fragrance materials.

### Background of the Invention

There is an ongoing need in the fragrance industry to provide new chemicals to give perfumers and other persons the ability to create new fragrances for perfumes, colognes and personal care products. Those with skill in the art appreciate how small differences in chemical structures can result in unexpected and significant differences in odor, notes and characteristics of molecules. These variations allow perfumers and other persons to apply new compounds in creating new fragrances. For example, benzene compounds that differ slightly in substituents possess completely different odor profiles [Ishikawa, et al., International Journal of Quantum Chemistry 79: 101-108 (2000)]. In the case of tert-butyl cyclohexanes, the odor is said to be dependent on the compounds' conformation and therefore analogs adopting same conformation possess similar odor. Accordingly, many trans-compounds are shown to share pronounced urine-perspiration-type odor, while the corresponding cis-compounds are odorless or at the most possess weak and undefinable flowery or woody odor. However, some other trans- and cis-tert-butyl cyclohexanes are shown to possess opposite sensory activities [Ohloff, et al., Helvetica Chimica Acta 66, Fasc. 5: 1343-1354 (1983)]. Thus, it is hard for those with skill in the art to predict a given structure would be effective in sensory activities. Identifying desirable fragrance chemicals continues to pose difficult challenges. EP0825166 relates to methyl substituted hexahydroindanols and their uses in perfumery. EP1262481 relates to naphthofurans and hydrogenated naphthofurans and their use in fragrances. US7176324 discloses hexahydroindan acetal and ketal compounds and their use in fragrances. US2004/110991 descirbes polyalkylbicylic chemical derivatives for in fragrances. US5002929 discloses derivatives of trimethylbicyclo-[4.3.0]-nonane which are useful as perfumes.

### Summary of the Invention

The present invention provides novel compounds, the unexpected advantageous use thereof in enhancing, improving or modifying the fragrance of perfumes, colognes, toilet waters, fabric care products, personal products and the like.

The present invention relates to novel octahydro-indenes represented by the formulas set forth below: 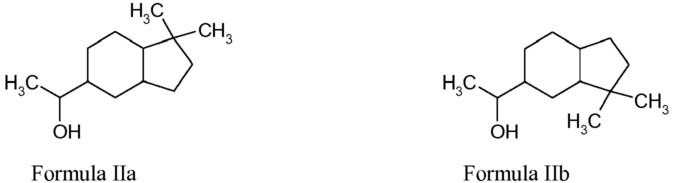 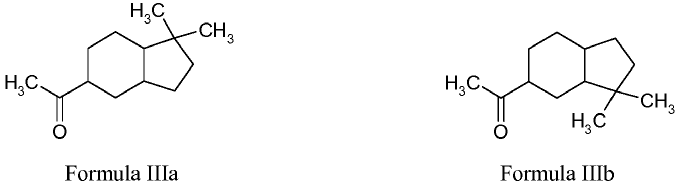 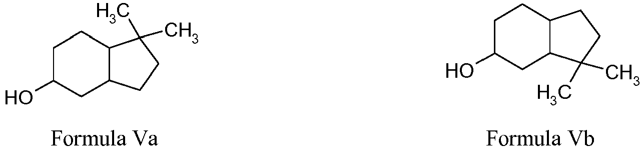 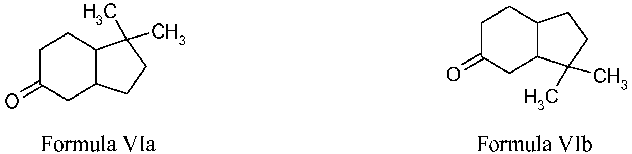 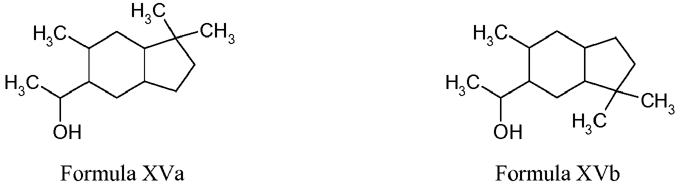
wherein Formula IIa represents 1-(1,1-dimethyl-octahydro-inden-5-yl)-ethanol;
Formula IIb represents 1-(3,3-dimethyl-octahydro-inden-5-yl)-ethanol;
Formula IIIa represents 1-(1,1-dimethyl-octahydro-inden-5-yl)-ethanone;
Formula IIIb represents 1-(3,3-dimethyl-octahydro-inden-5-yl)-ethanone;
Formula Va represents 1,1-dimethyl-octahydro-inden-5-ol;
Formula Vb represents 3,3-dimethyl-octahydro-inden-5-ol;
Formula VIa represents 1,1-dimethyl-octahydro-inden-5-one;
Formula VIb represents 3,3-dimethyl-octahydro-inden-5-one;
Formula XVa represents 1-(1,1,6-trimethyl-octahydro-inden-5-yl)-ethanol;
Formula XVb represents 1-(3,3,6-trimethyl-octahydro-inden-5-yl)-ethanol;

For reference only, the following octahydro-indenes are represented by the formulas set forth below: 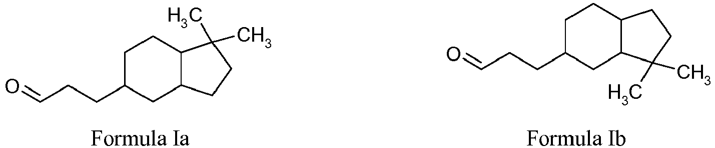 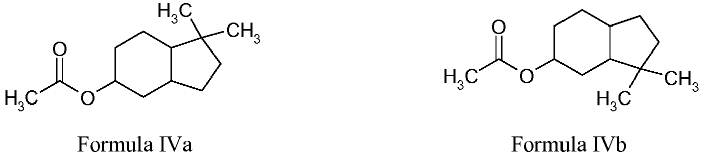 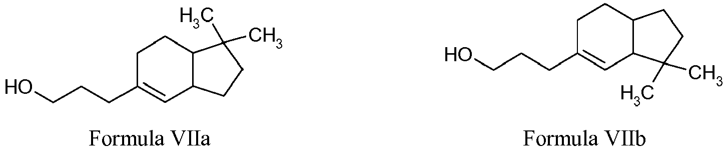 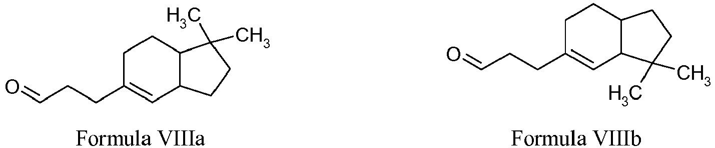 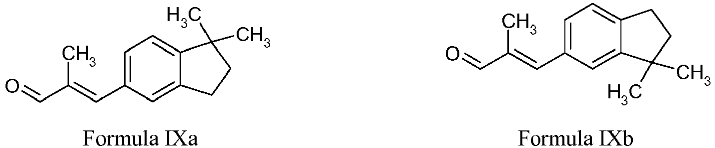 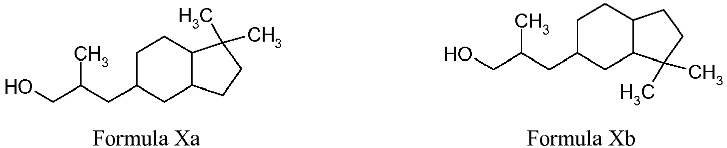 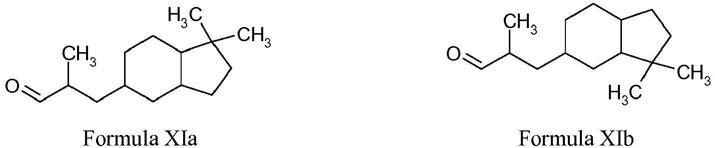 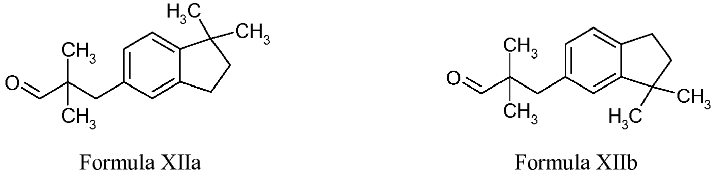 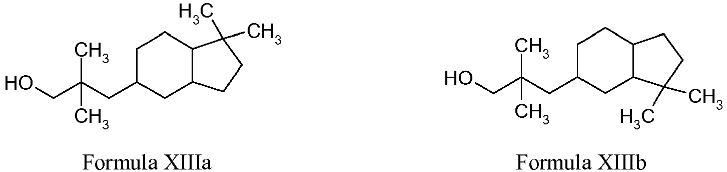 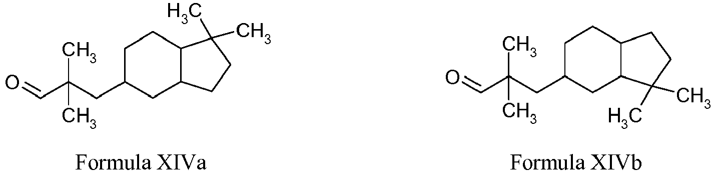 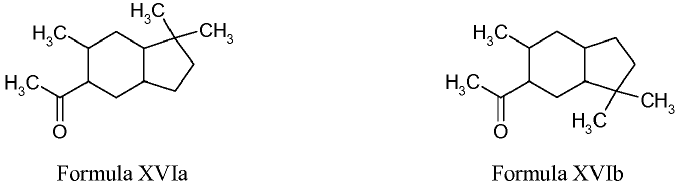 wherein
Formula Ia represents 3-(1,1-dimethyl-octahydro-inden-5-yl)-propanal;
Formula Ib represents 3-(3,3- dimethyl-octahydro-inden-5-yl)- propanal;
Formula IVa represents 1,1-dimethyl-octahydro-inden-5-yl acetate;
Formula IVb represents 3,3-dimethyl-octahydro-inden-5-yl acetate;
Formula VIIa represents 3-(1,1-dimethyl-2,3,3a,6,7,7a-hexahydro-1H-inden-5-yl)-propan-1-ol;
Formula VIIb represents 3-(3,3-dimethyl-2,3,3a,6,7,7a-hexahydro-1H-inden-5-yl)-propan-1-ol;
Formula VIIIa represents 3-(1,1-dimethyl-2,3,3a,6,7,7a-hexahydro-1H-inden-5-yl)-propanal;
Formula VIIIb represents 3-(3,3-dimethyl-2,3,3a,6,7,7a-hexahydro-1H-inden-5-yl)-propanal;
Formula IXa represents (E)-3-(1,1-dimethyl-indan-5-yl)-2-methyl-propenal;
Formula IXb represents (E)-3-(3,3-dimethyl-indan-5-yl)-2-methyl-propenal;
Formula Xa represents 3-(1,1-dimethyl-octahydro-inden-5-yl)-2-methyl-propan-1-ol;
Formula Xb represents 3-(3,3-dimethyl-octahydro-inden-5-yl)-2-methyl-propan-1-ol;
Formula XIa represents 3-(1,1-dimethyl-octahydro-inden-5-yl)-2-methyl-propanal;
Formula XIb represents 3-(3,3-dimethyl-octahydro-inden-5-yl)-2-methyl-propanal;
Formula XIIa represents 3-(1,1-dimethyl-indan-5-yl)-2,2-dimethyl-propanal;
Formula XIIb represents 3-(3,3-dimethyl-indan-5-yl)-2,2-dimethyl-propanal;
Formula XIIIa represents 3-(1,1-dimethyl-octahydro-inden-5-yl)-2,2-dimethyl-propan-1-ol;
Formula XIIIb represents 3-(3,3-dimethyl-octahydro-inden-5-yl)-2,2-dimethyl-propan-1-ol;
Formula XIVa represents 3-(1,1-dimethyl-octahydro-inden-5-yl)-2,2-dimethyl-propionaldehyde;
Formula XIVb represents 3-(1,1-dimethyl-octahydro-inden-5-yl)-2,2-dimethyl-propionaldehyde;
Formula XVIa represents 1-(1,1,6-trimethyl-octahydro-inden-5-yl)-ethanone; and
Formula XVIb represents 1-(3,3,6-trimethyl-octahydro-inden-5-yl)-ethanone.

Specifically, in a first aspect the present invention provides a compound selected from the group consisting of:
1-(1,1-dimethyl-octahydro-inden-5-yl)-ethanol;
1-(3,3-dimethyl-octahydro-inden-5-yl)-ethanol;
1-(1,1-dimethyl-octahydro-inden-5-yl)-ethanone;
1-(3,3-dimethyl-octahydro-inden-5-yl)-ethanone;
1,1-dimethyl-octahydro-inden-5-ol;
3,3-dimethyl-octahydro-inden-5-ol;
1,1-dimethyl-octahydro-inden-5 -one;
3,3-dimethyl-octahydro-inden-5-one;
1-(1,1,6-trimethyl-octahydro-inden-5-yl)-ethanol;
1-(3,3,6-trimethyl-octahydro-inden-5-yl)-ethanol; and
a mixture thereof.

In another aspect, the invention provides a method of counteracting malodor in an air space or a substrate comprising the step of introducing a malodor counteracting effective amount of a compound of the invention.

In another aspect, the invention provides a fragrance formulation comprising an olfactory acceptable amount of a compound selected from the group consisting of
1-(1,1-dimethyl-octahydro-inden-5-yl)-ethanol;
1-(3,3-dimethyl-octahydro-inden-5-yl)-ethanol;
1-(1,1-dimethyl-octahydro-inden-5-yl)-ethanone;
1-(3,3-dimethyl-octahydro-inden-5-yl)-ethanone;
1,1-dimethyl-octahydro-inden-5-ol;
3,3-dimethyl-octahydro-inden-5-ol;
1,1-dimethyl-octahydro-inden-5-one;
3,3-dimethyl-octahydro-inden-5-one;
1-(1,1,6-trimethyl-octahydro-inden-5-yl)-ethanol;
1-(3,3,6-trimethyl-octahydro-inden-5-yl)-ethanol; and
a mixture thereof.

In another aspect, the invention provides a method of improving, enhancing or modifying a fragrance formulation through the addition of an olfactory acceptable amount of a compound selected from the group consisting of:
1-(1,1-dimethyl-octahydro-inden-5-yl)-ethanol;
1-(3,3-dimethyl-octahydro-inden-5-yl)-ethanol;
1-(1,1-dimethyl-octahydro-inden-5-yl)-ethanone;
1-(3,3-dimethyl-octahydro-inden-5-yl)-ethanone;
1,1-dimethyl-octahydro-inden-5-ol;
3,3-dimethyl-octahydro-inden-5-ol;
1,1-dimethyl-octahydro-inden-5-one;
3,3-dimethyl-octahydro-inden-5-one;
1-(1,1,6-trimethyl-octahydro-inden-5-yl)-ethanol;
1-(3,3,6-trimethyl-octahydro-inden-5-yl)-ethanol; and a mixture thereof.

In another aspect, the invention provides a fragrance product containing an olfactory acceptable amount of a compound of the invention.

Embodiments of the present invention will be apparent by reading the following specification.

### Detailed Description of the Invention

The compounds of the present invention can be prepared according to the reaction steps detailed in the Examples. Materials and catalysts were purchased from Sigma-Aldrich Chemical Company unless noted otherwise. Those with skill in the art will further recognize that some of the compounds of the present invention have a number of chiral centers, thereby providing numerous isomers of the claimed compounds. It is intended herein that the compounds described herein include isomeric mixtures of such compounds, as well as those isomers that may be separated using techniques known to those having skill in the art. Suitable techniques include, for example, distillation and chromatography such as high performance liquid chromatography, referred to as HPLC, particularly silica gel chromatograph, and gas chromatography trapping known as GC trapping. Yet, commercial products are mostly offered as isomeric mixtures.

The compounds of the present invention are surprisingly found to possess powerful and complex fragrance effect such as, for example, floral and muguet notes.

The use of the compounds of the present invention is widely applicable in current perfumery products, including the preparations of perfumes and colognes, the perfuming of personal care products such as soaps, shower gels, and hair care products, fabric care products, air fresheners, and cosmetic preparations. The present invention can also be used to perfume cleaning agents, such as, but not limited to detergents, dishwashing materials, scrubbing compositions, window cleaners and the like. In these preparations, the compounds of the present invention can be used alone or in combination with other perfuming compositions, solvents, adjuvants and the like. The nature and variety of the other ingredients that can also be employed are known to those with skill in the art. Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in US Pat. No. 4,534,891. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

The compounds of the present invention can be used in combination with a complementary fragrance compound. The term "complementary fragrance compound" as used herein is defined as a fragrance compound selected from the group consisting of 2-[(4-methylphenyl)methylene]-heptanal (Acalea), iso-amyl oxyacetic acid allylester (Allyl Amyl Glycolate), (3,3-dimethylcyclohexyl)ethyl ethyl propane-1,3-dioate (Applelide), octahydro-4,7-methano-1H-indene-5-acetaldehyde; (E/Z)-1-ethoxy-1-decene (Arctical), 2-ethyl-4-(2,2,3-trimethyl-3-cyclo-penten-1-yl)-2-buten-1-ol (Bacdanol), 2-methyl-3-[(1,7,7-trimethylbicyclo[2.2.1]hept-2-yl)oxy] exo-1-propanol (Bornafix), 1,2,3,5,6,7-hexahydro-1,1,2,3,3-pentamethyl-4H-inden-4-one (Cashmeran), trimethylcyclopentenylmethyloxabicyclooctane (Cassiffix), 1,1-dimethoxy-3,7-dimethyl-2,6-octadiene (Citral DMA), 3,7-dimethyl-6-octen-1-ol (Citronellol), 3A,4,5,6,7,7A-hexahydro-4,7-methano-1*H*-inden-5/6-yl acetate (Cyclacet), 3A,4,5,6,7,7A-hexahydro-4,7-methano-1*H*-inden-5/6-yl propinoate (Cyclaprop), 3A,4,5,6,7,7A-hexahydro-4,7-methano-1G-inden-5/6-yl butyrate (Cyclobutanate), 1-(2,6,6-trimethyl-3-cyclohexen-1-yl)-2-buten-1-one (Delta Damascone), (1S)-1-[(1R)-3,3-dimethylcyclohexyl]ethyl ethyl malonate, 4-ethyloctanal, 3-(4-ethylphenyl)-2,2-dimethyl propanenitrile (Fleuranil), 3-(O/P-ethylphenyl) 2,2-dimethyl propionaldehyde (Floralozone), tetrahydro-4-methyl-2-(2-methylpropyl)-2H-pyran-4-ol (Floriffol), 1,3,4,6,7,8-hexahydro-4,6,6,7,8,8-hexamethylcyclopenta-gamma-2-benzopyran (Galaxolide), 1-(5,5-dimethyl-1-cyclohexen-1-yl)pent-4-en-1-one (Galbascone), E/Z-3,7-dimethyl-2,6-octadien-1-yl acetate (Geranyl Acetate), α-methyl-1,3-benzodioxole-5-propanal (Helional), 1-(2,6,6-trimethyl-2-cyclohexen-1-yl)-1,6-heptadien-3-one (Hexalon), (Z)-3-hexenyl-2-hydroxybenzoate (Hexenyl Salicylate, CIS-3), 4-(2,6,6-trimethyl-2-cyclohexen-1-yl)-3-buten-2-one (Ionone α), 1-(1,2,3,4,5,6,7,8-octahydro-2,3,8,8-tetramethyl-2-naphthalenyl)-ethan-1-one (Iso E Super), methyl 3-methylcyclohexanecarboxylate, methyl 3-oxo-2-pentylcyclopentaneacetate (Kharismal), 2,2,4-trimethyl-4-phenyl-butanenitrile (Khusinil), 3,4,5,6,6-pentamethylhept-3-en-2-one (Koavone), 3/4-(4-hydroxy-4-methyl-pentyl) cyclohexene-1-carboxaldehyde (Lyral), 3-methyl-4-(2,6,6-trimethyl-2-cyclohexen-1-yl)-3-buten-2-one (Methyl Ionone γ), 1-(2,6,6-trimethyl-2-cyclohexen-1-yl) pent-1-en-3-one (Methyl Ionone α Extra, Methyl Ionone N), [(Z)-hex-3-enyl] cyclopropanecarboxylate, 3-methyl-4-phenylbutan-2-ol (Muguesia), cyclopentadec-4-en-1-one (Musk Z4), 3,3,4,5,5-pentamethyl-11,13-dioxatricyclo[7.4.0.0<2,6>]tridec-2(6)-ene (Nebulone), 3,7-dimethyl-2,6-octadien-1-yl acetate (Neryl Acetate), 3,7-dimethyl-1,3,6-octatriene (Ocimene), dec-6 or 7 or 8-enal, 2,2,6,6,7,8,8-heptamethyl-4,5,6,7,8,8B-hexahydro-3AH-indeno[4,5-D][1,3]dioxole, cis-1-(1,1-dimethylpropyl)-4-ethoxy cyclohexane, ortho-tolylethanol (Peomosa), 3-methyl-5-phenylpentanol (Phenoxanol), 1-methyl-4-(4-methyl-3-pentenyl) cyclohex-3-ene-1-carboxaldehyde (Precyclemone B), 4-methyl-8-methylene-2-adamantanol (Prismantol), 2-ethyl-4-(2,2,3-trimethyl-3-cyclopenten-1-yl)-2-buten-1-ol (Sanjinol), 2-methyl-4-(2,2,3-trimethyl-3-cyclopenten-1-yl)-2-buten-1-ol (Santaliff), 7,7,8,9,9-pentamethyl-6,7,8,9-tetrahydro-5H-cyclopenta[H]quinazoline, 3-[cis-4-(2-methylpropyl)cyclohexyl]propanal, 4-(heptyloxy)-3-methylbutanal, Terpineol, 2,4-dimethyl-3-cyclohexene-1-carboxaldehyde (Triplal), decahydro-2,6,6,7,8,8-hexamethyl-2H-indeno[4,5-B]furan (Trisamber), ethyl bicyclo[2.2.1]hept-5-ene-2-carboxylate, 3,4,5-trimethyloctahydro-1H-4,7-methanoinden-5-ol, 2-tert-butylcyclohexyl acetate (Verdox), (3E)-4-methyldec-3-en-5-one , 4-tert-butylcyclohexyL acetate (Vertenex), acetyl cedrene (Vertofix), 3,6/4,6-dimethylcyclohex-3-ene-1-carboxaldehyde (Vertoliff) and (3Z)-1-[(2-methyl-2-propenyl)oxy]-3-hexene (Vivaldie).

Complexity of odor notes refers to the presence of multiple and/or mixed but defined odors rather than a single note or a few easily identifiable notes. High levels of complexity are also assigned to compounds that possess ambiguous and somehow hard-to-define notes because of direct contribution or the many olfactive combinations of odors produced. Fragrance materials of high level complexity are considered having unusual and high quality.

The terms "fragrance formulation", "fragrance composition", and "perfume composition" mean the same and refer to a consumer composition that is a mixture of compounds including, for example, alcohols, aldehydes, ketones, esters, ethers, lactones, nitriles, natural oils, synthetic oils, and mercaptans, which are admixed so that the combined odors of the individual components produce a pleasant or desired fragrance. The fragrance formulation of the present invention is a consumer composition comprising a compound of the present invention. The fragrance formulation of the present invention may comprise a compound of the present invention and further a complementary fragrance compound as defined above.

The term "fragrance product" means a consumer product containing a fragrance ingredient that adds fragrance or masks malodor. Fragrance products may include, for example, perfumes, colognes, bar soaps, liquid soaps, shower gels, foam baths, cosmetics, skin care products such as creams, lotions and shaving products, hair care products for shampooing, rinsing, conditioning, bleaching, coloring, dyeing and styling, deodorants and antiperspirants, feminine care products such as tampons and feminine napkins, baby care products such as diapers, bibs and wipes, family care products such as bath tissues, facial tissues, paper handkerchiefs or paper towels, fabric products such as fabric softeners and fresheners, air care products such as air fresheners and fragrance delivery systems, cosmetic preparations, cleaning agents and disinfectants such as detergents, dishwashing materials, scrubbing compositions, glass and metal cleaners such as window cleaners, countertop cleaners, floor and carpet cleaners, toilet cleaners and bleach additives, washing agents such as all-purpose, heavy duty, and hand washing or fine fabric washing agents including laundry detergents and rinse additives, dental and oral hygiene products such as toothpastes, tooth gels, dental flosses, denture cleansers, denture adhesives, dentifrices, tooth whitening and mouthwashes, health care and nutritional products and food products such as snack and beverage products. The fragrance product of the present invention is a consumer product that contains a compound of the present invention. The fragrance product of the present invention may contain a compound of the present invention and further a complementary fragrance compound as defined above.

The term "improving" in the phrase "improving, enhancing or modifying a fragrance formulation" is understood to mean raising the fragrance formulation to a more desirable character. The term "enhancing" is understood to mean making the fragrance formulation greater in effectiveness or providing the fragrance formulation with an improved character. The term "modifying" is understood to mean providing the fragrance formulation with a change in character.

The term "olfactory acceptable amount" is understood to mean the amount of a compound in a fragrance formulation, wherein the compound will contribute its individual olfactory characteristics. However, the olfactory effect of the fragrance formulation will be the sum of effect of each of the fragrance ingredients. Thus, the compounds of the present invention can be used to improve or enhance the aroma characteristics of the fragrance formulation, or by modifying the olfactory reaction contributed by other ingredients in the formulation. The olfactory acceptable amount may vary depending on many factors including other ingredients, their relative amounts and the olfactory effect that is desired.

The amount of the compounds of the present invention employed in a fragrance formulation varies from about 0.005 to about 70 weight percent, preferably from 0.05 to about 50 weight percent, more preferably from about 0.5 to about 25 weight percent, and even more preferably from about 1 to about 10 weight percent. Those with skill in the art will be able to employ the desired amount to provide desired fragrance effect and intensity. In addition to the compounds of the present invention, other materials can also be used in conjunction with the fragrance formulation to encapsulate and/or deliver the fragrance. Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica.

When used in a fragrance formulation these ingredients provide additional notes to make a fragrance formulation more desirable and noticeable, and add the perception of value. The odor qualities found in these materials assist in beautifying and enhancing the finished accord as well as improving the performance of the other materials in the fragrance.

In addition, the compounds of the present invention are also surprisingly found to provide superior ingredient performance and possess unexpected advantages in malodor counteracting applications such as body perspiration, environmental odor such as mold and mildew, bathroom, and etc. The compounds of the present invention substantially eliminate the perception of malodors and/or prevent the formation of such malodors, thus, can be utilized with a vast number of functional products.

Examples of the functional products are provided herein to illustrate the various aspects of the present invention. However, they do not intend to limit the scope of the present invention. The functional products may include, for example, a conventional room freshener (or deodorant) composition such as room freshener sprays, an aerosol or other spray, fragrance diffusers, a wick or other liquid system, or a solid, for instance candles or a wax base as in pomanders and plastics, powders as in sachets or dry sprays or gels, as in solid gel sticks, clothes deodorants as applied by washing machine applications such as in detergents, powders, liquids, whiteners or fabric softeners, fabric refreshers, linen sprays, closet blocks, closet aerosol sprays, or clothes storage areas or in dry cleaning to overcome residual solvent notes on clothes, bathroom accessories such as paper towels, bathroom tissues, sanitary napkins, towellets, disposable wash cloths, disposable diapers, and diaper pail deodorants, cleansers such as disinfectants and toilet bowl cleaners, cosmetic products such as antiperspirant and deodorants, general body deodorants in the form of powders, aerosols, liquids or solid, or hair care products such as hair sprays, conditioners, rinses, hair colors and dyes, permanent waves, depilatories, hair straighteners, hair groom applications such as pomade, creams and lotions, medicated hair care products containing such ingredients as selenium sulphide, coal tar or salicylates, or shampoos, or foot care products such as foot powders, liquids or colognes, after shaves and body lotions, or soaps and synthetic detergents such as bars, liquids, foams or powders, odor control such as during manufacturing processes, such as in the textile finishing industry and the printing industry (inks and paper), effluent control such as in processes involved in pulping, stock yard and meat processings, sewage treatment, garbage bags, or garbage disposal, or in product odor control as in textile finished goods, rubber finished goods or car fresheners, agricultural and pet care products such as dog and hen house effluents and domestic animal and pet care products such as deodorants, shampoo or cleaning agents, or animal litter material and in large scale closed air systems such as auditoria, and subways and transport systems.

Thus, it will be seen that the composition of the invention is usually one in which the malodor counteractant is present together with a carrier by means of which or from which the malodor counteractant can be introduced into air space wherein the malodor is present, or a substrate on which the malodor has deposited. For example, the carrier can be an aerosol propellant such as a chlorofluoro-methane, or a solid such as a wax, plastics material, rubber, inert powder or gel. In a wick-type air freshener, the carrier is a substantially odorless liquid of low volatility. In several applications, a composition of the invention contains a surface active agent or a disinfectant, while in others, the malodor counteractant is present on a fibrous substrate. In many compositions of the invention there is also present a fragrance component which imparts a fragrance to the composition. The fragrances stated above can all be employed.

Malodor counteracting effective amount is understood to mean the amount of the inventive malodor counteractant employed in a functional product that is organoleptically effective to abate a given malodor while reducing the combined intensity of the odor level, wherein the given malodor is present in air space or has deposited on a substrate. The exact amount of malodor counteractant agent employed may vary depending upon the type of malodor counteractant, the type of the carrier employed, and the level of malodor counteractancy desired. In general, the amount of malodor counteractant agent present is the ordinary dosage required to obtain the desired result. Such dosage is known to the skilled practitioner in the art. In a preferred embodiment, when used in conjunction with malodorous solid or liquid functional products, e.g., soap and detergent, the compounds of the present invention may be present in an amount ranging from about 0.005 to about 50 weight percent, preferably from about 0.01 to about 20 weight percent, and more preferably from about 0.05 to about 5 weight percent, and when used in conjunction with malodorous gaseous functional products, the compounds of the present invention may be present in an amount ranging from about 0.1 to 10 mg per cubic meter of air.

The following are provided as specific embodiments of the present invention, unless otherwise marked as reference examples. Other modifications of this invention will be readily apparent to those skilled in the art. Such modifications are understood to be within the scope of this invention. As used herein all percentages are weight percent unless otherwise noted, ppm is understood to stand for parts per million, L is understood to be liter, mL is understood to be milliliter, g is understood to be gram, mol is understood to be mole, psi is understood to be pounds per square inch and mmHg be millimeters (mm) of mercury (Hg). IFF as used in the examples is understood to mean International Flavors & Fragrances Inc., New York, NY, USA.

### REFERENCE EXAMPLE I

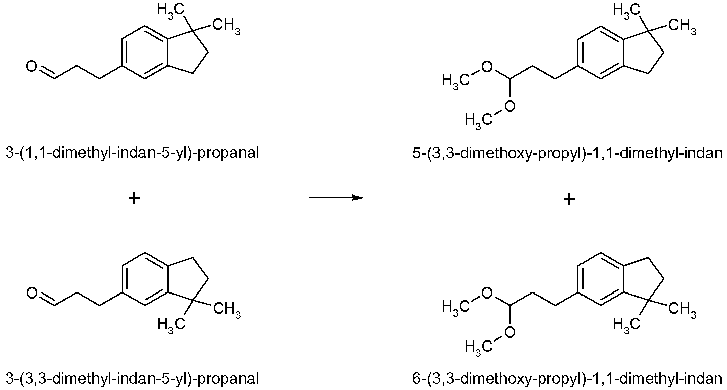

**Preparation of 5-(3,3-Dimethoxy-propyl)-1,1-dimethyl-indan and 6-(3,3-Dimethoxy-propyl)-1,1-dimethyl-indan:** The mixture of 3-(1,1-dimethyl-indan-5-yl)-propanal and 3-(3,3-dimethyl-indan-5-yl)-propanal in a weight ratio of 40:60 (*See,* US Pat. No. 5,552,379) (150 g, 0.74 mol) was dissolved in trimethyl orthoformate (HC(OCH₃)₃) (250 g) and methanol (50 g) and cooled to -10 °C. Hydrochloric acid (HCl) (12 M, 1 mL) was added and the reaction rapidly exothermed to 40 °C. Sodium acetate (CH3COONa) (20 g) was added. The resulting reaction mixture was distilled to provide the mixture of 5-(3,3-dimethoxy-propyl)-1,1-dimethyl-indan and 6-(3,3-dimethoxy-propyl)-1,1-dimethyl-indan in a weight ratio of 40:60 (170 g).

The mixture of 5-(3,3-dimethoxy-propyl)-1,1-dimethyl-indan and 6-(3,3-dimethoxy-propyl)-1,1-dimethyl-indan was described as having chemical, kerosene-like, muguet and spicy notes.

The mixture of 5-(3,3-dimethoxy-propyl)-1,1-dimethyl-indan and 6-(3,3-dimethoxy-propyl)-1,1-dimethyl-indan possessed the NMR spectral characteristics of:
¹H NMR (CDCl₃, 500 MHz): 6.95-7.13 (m, 3H), 4.38 (q, J=5.6 Hz, 1H), 3.33 (s, 6H), 2.79-2.90 (m, 2H), 2.60-2.69 (m, 2H), 1.87-1.95 (m, 4H), 1.24 (s, 6H)

### REFERENCE EXAMPLE II

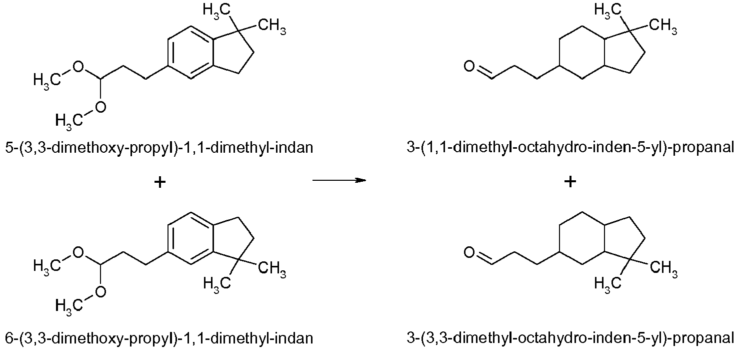

**Preparation of 3-(1,1-Dimethyl-octahydro-inden-5-yl)-propanal (Formula Ia) and 3-(3,3-Dimethyl-octahydro-inden-5-yl)-propanal (Formula Ib):** The mixture of 5-(3,3-dimethoxy-propyl)-1,1-dimethyl-indan and 6-(3,3-dimethoxy-propyl)-1,1-dimethyl-indan (obtained as above in EXAMPLE I) (108 g, 435 mmol) in isopropanol (CH₃CHOHCH₃) (200 mL) was hydrogenated over ruthenium (IV) oxide (RuO₂) at 60 °C under 400 psi (2757.90 kPa) hydrogen (H₂) for 8 hours. The reaction was cooled and vented to atmospheric pressure. The resulting mixture was filtered through Celite® and evaporated to dryness. The crude was dissolved in methyl ethyl ketone (MEK) (CH₃C(O)CH₂CH₃) (200 mL). To the crude solution was added water (50 mL) and HCl (12 M, 1 mL) and the resulting mixture was heated to reflux for 1 hour. The reaction was then cooled and toluene (20 mL) was added. The organic layer was separated and evaporated to dryness. Further distillation provided the mixture of 3-(1,1-dimethyl-octahydro-inden-5-yl)-propanal (Formula Ia) and 3-(3,3-dimethyl-octahydro-inden-5-yl)-propanal (Formula Ib) in a weight ratio of 40:60 (85 g).

The mixture of Formula Ia and Ib was described as having floral, muguet, ozonic, orris and waxy notes. Appeared chemical and harsh.

The mixture of Formula Ia and Ib possessed the NMR spectral characteristics of:
¹H NMR (500 MHz, CDCl₃): 9.70-9.74 (m, 1H), 0.54-2.20 (m, 17H), 0.93 (s, 3H), 0.88 (s, 3H)

### REFERENCE EXAMPLE III

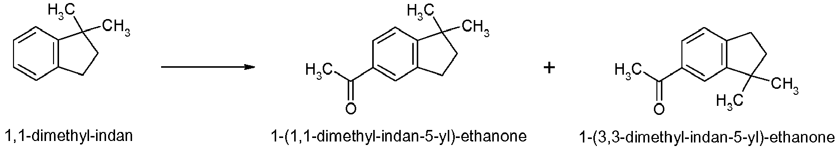

**Preparation of 1-(1,1-Dimethyl-indan-5-yl)-ethanone and 1-(3,3-Dimethyl-indan-5-yl)-ethanone:** Aluminum chloride (AlCl₃) (408 g, 3.1 mol) was dissolved in dichloromethane (CH₂Cl₂) (1 L) and cooled to 0 °C. Acetyl chloride (CH₃COCl) (240 g, 3.1 mol) was fed in over 30 minutes. While the temperature was maintained at 0 °C, 1,1-dimethyl-indan (448 g, 3.1 mol) was added and the mixture was stirred for 2 hours. The resulting mixture was poured into ice (5 Kg). The organic layer was then separated and distilled to provide a clear oil comprising the mixture of 1-(1,1-dimethyl-indan-5-yl)-ethanone and 1-(3,3-dimethyl-indan-5-yl)-ethanone in a weight ratio of 40:60 (420 g).

The mixture of 1-(1,1-dimethyl-indan-5-yl)-ethanone and 1-(3,3-dimethyl-indan-5-yl)-ethanone was described as having chemical, kerosene, gassy and spicy notes.

1-(1,1-Dimethyl-indan-5-yl)-ethanone:
¹H NMR (CDCl₃, 500 MHz): 7.77-7.81 (m, 1H), 7.73-7.76 (m, 1H), 7.24 (d, J=8.5 Hz, 1H), 2.92 (t, J=7.3 Hz, 2H), 2.56 (s, 3H), 1.95 (t, J=7.3 Hz, 2H), 1.27 (s, 3H), 1.26 (s, 3H)

1-(3,3-Dimethyl-indan-5-yl)-ethanone:
¹H NMR (CDCl₃, 500 MHz): 7.77-7.81 (m, 1H), 7.73-7.76 (m, 1H), 7.19 (d, J=8.5 Hz, 1H), 2.92 (t, J=7.3 Hz, 2H), 2.58 (s, 3H), 1.95 (t, J=7.3 Hz, 2H), 1.27 (s, 3H), 1.26 (s, 3H)

### EXAMPLE IV

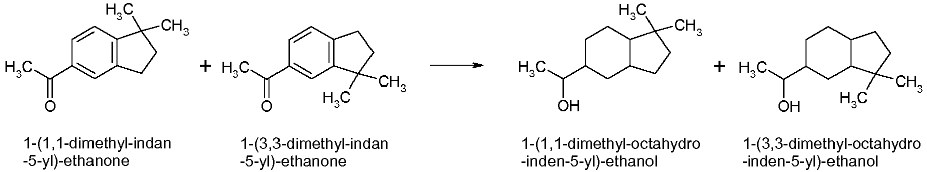

**Preparation of 1-(1,1-Dimethyl-octahydro-inden-5-yl)-ethanol (Formula IIa) and 1-(3,3-Dimethyl-octahydro-inden-5-yl)-ethanol (Formula IIb):** The mixture of 1-(1,1-dimethyl-indan-5-yl)-ethanone and 1-(3,3-dimethyl-indan-5-yl)-ethanone (obtained as above in EXAMPLE III) (535 g, 2.8 mol) was hydrogenated over ruthenium(IV) oxide (3 g, 8 mmol) at 50 °C under 2757. 90 kPa (400 psi) H₂ for 12 hours. The reaction was cooled and vented to atmospheric pressure to provide the mixture of 1-(1,1-dimethyl-octahydro-inden-5-yl)-ethanol (Formula IIa) and 1-(3,3-dimethyl-octahydro-inden-5-yl)-ethanol (Formula IIb) in a weight ratio of 40:60 (475 g).

The mixture of Formula IIa and IIb was described as having strong floral, spicy and woody notes with fruity, citrusy, green, herbaceous and particularly fresh and watery characters.

1-(1,1-Dimethyl-octahydro-inden-5-yl)-ethanol:
¹H NMR (CDCl₃, 400 MHz): 3.47-3.74 (m, 1H), 0.57-2.55 (m, 14H), 1.15 (d, J=6.8Hz, 3H), 0.94 (s, 3H), 0.92 (s, 3H)

1-(3,3-Dimethyl-octahydro-inden-5-yl)-ethanol
¹H NMR (CDCl₃, 400 MHz): 3.47-3.74 (m, 1H), 0.57-2.55 (m, 14H), 1.17 (d, J=6.8Hz, 3H), 0.98 (s, 3H), 0.96 (s, 3H)

### EXAMPLE V

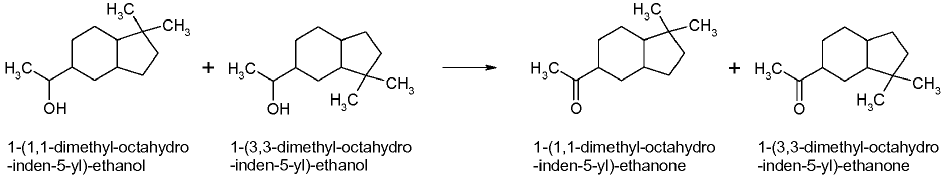

**Preparation of 1-(1,1-Dimethyl-octahydro-inden-5-yl)-ethanone (Formula IIIa) and 1-(3,3-Dimethyl-octahydro-inden-5-yl)-ethanone (Formula IIIb):** The mixture of 1-(1,1-dimethyl-octahydro-inden-5-yl)-ethanol and 1-(3,3-dimethyl-octahydro-inden-5-yl)-ethanol (obtained as above in EXAMPLE IV) (425 g, 2.2 mol) was placed in a reaction vessel with PriCat CZ 30/18P dehydrogenation catalyst (Johnson Matthey Process Technologies) (25 g) and mineral oil (150 mL). The reaction was slowly heated to 175 °C, aged at this temperature for 10 hours and cooled. Further distillation provided a clear oil comprising the mixture of 1-(1,1-dimethyl-octahydro-inden-5-yl)-ethanone (Formula IIIa) and 1-(3,3-dimethyl-octahydro-inden-5-yl)-ethanone (Formula IIIb) in a weight ratio of 40:60 (375 g).

The mixture of Formula IIIa and IIIb was described as having strong musky and woody notes with powdery and sweet characters.

The mixture of Formula IIIa and IIIb possessed the NMR spectral characteristics of:
¹H NMR (CDCl₃, 500 MHz): 2.14-2.57 (m, 2H), 2.10 (s, 3H), 0.95-1.93 (m, 11H), 0.79-0.95 (m, 6H)

### REFERENCE EXAMPLE VI

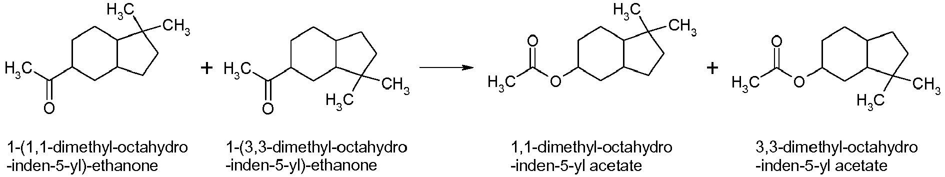

**Preparation of 1,1-Dimethyl-octahydro-inden-5-yl Acetate (Formula IVa) and 3,3-Dimethyl-octahydro-inden-5-yl Acetate (Formula IVb):** The mixture of 1-(1,1-dimethyl-octahydro-inden-5-yl)-ethanone and 1-(3,3-dimethyl-octahydro-inden-5-yl)-ethanone (obtained as above in EXAMPLE V) (223 g, 1.1mol) was dissolved in toluene (1 L) and heated to 50 °C. To the mixture was fed peracetic acid (CH₃CO₃H) (32%) (273 g, 1.1 mol) over 2 hours. The reaction was aged for additional 4 hours, cooled and washed with aqueous sodium bisulfite solution (NaHSO₃) (1 M) (500 mL). The resulting organic layer was distilled to provide the mixture of 1,1-dimethyl-octahydro-inden-5-yl acetate (Formula IVa) and 3,3-dimethyl-octahydro-inden-5-yl acetate (Formula IVb) in a weight ratio of 40:60 (196 g).

The mixture of Formula IVa and IVb was described as having smoky and strong burnt notes.

The mixture of Formula IVa and IVb possessed the NMR spectral characteristics of:
¹H NMR (CDCl₃, 500 MHz): 4.54-5.02 (m, 1H), 2.27-2.59 (m, 1H), 1.96-2.06 (m, 3H), 0.97-1.95 (m, 11H), 0.86-0.96 (m, 6H)

### EXAMPLE VII

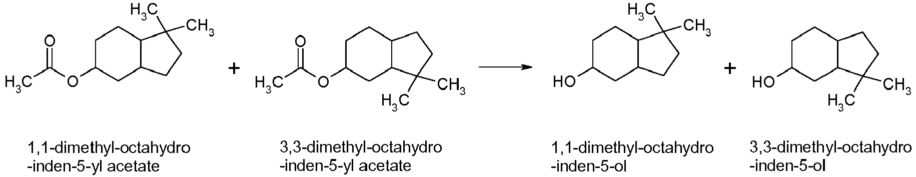

**Preparation of 1,1-Dimethyl-octahydro-inden-5-ol (Formula Va) and 3,3-Dimethyl-octahydro-inden-5-ol (Formula Vb):** The mixture of 1,1-dimethyl-octahydro-inden-5-yl acetate and 3,3-dimethyl-octahydro-inden-5-yl acetate (obtained as above in EXAMPLE VI) (185 g, 0.88 mol) was stirred at 80 °C in ethanol (500 mL) with potassium hydroxide (KOH) (99 g, 1.8 mol) for 6 hours. The reaction was cooled and acidified to pH 5 with acetic acid (CH₃COOH). Water (1 L) and toluene (1 L) were subsequently added. The resulting toluene layer was removed and distilled to provide the mixture of 1,1-dimethyl-octahydro-inden-5-ol (Formula Va) and 3,3-dimethyl-octahydro-inden-5-ol (Formula Vb) in a weight ratio of 40:60 (140 g).

The mixture of Formula Va and Vb was described as having strong animalic and leathery notes with clean and herbaceous characters.

The mixture of Formula Va and Vb possessed the NMR spectral characteristics of:
¹H NMR (CDCl₃, 400 MHz): 3.34-4.01 (m, 1H), 2.56-2.99 (m, 1H), 2.21-2.56 (m, 1H), 1.15-1.99 (m, 10H), 1.01-2.00 (m, 1H), 0.81-0.98 (m, 6H)

### EXAMPLE VIII

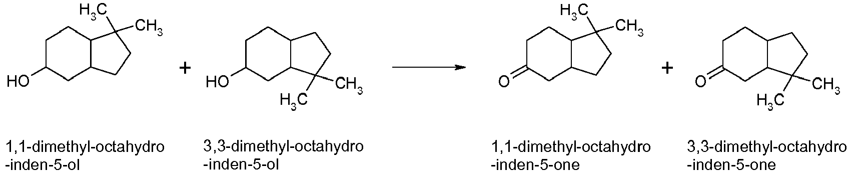

**Preparation of 1,1-Dimethyl-octahydro-inden-5-one (Formula VIa) and 3,3-Dimethyl-octahydro-inden-5-one (Formula VIb):** The mixture of 1,1-dimethyl-octahydro-inden-5-one (Formula VIa) and 3,3-dimethyl-octahydro-inden-5-one (Formula VIb) in a weight ratio of 40:60 (45 g) was prepared similarly according to EXAMPLE V using the mixture of 1,1-dimethyl-octahydro-inden-5-ol and 3,3-dimethyl-octahydro-inden-5-ol (obtained as above in EXAMPLE VII) (62 g).

The mixture of Formula VIa and VIb was described as having woody, strong leathery and herbaceous notes.

1,1-Dimethyl-octahydro-inden-5-one:
¹H NMR (CDCl₃, 400 MHz): 1.24-2.63 (m, 12H), 0.96 (s, 3H), 0.0.86 (s, 3H)

3,3-Dimethyl-octahydro-inden-5-one:
¹H NMR (CDCl₃, 400 MHz): 1.24-2.63 (m, 12H), 1.00 (s, 3H), 0.92 (s, 3H)

### REFERENCE EXAMPLE IX

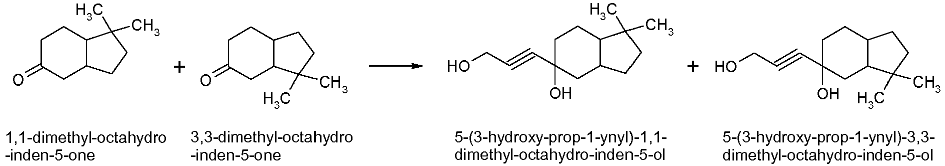

**Preparation of 5-(3-Hydroxy-prop-1-ynyl)-1,1-dimethyl-octahydro-inden-5-ol and 5-(3-Hydroxy-prop-1-ynyl)-3,3-dimethyl-octahydro-inden-5-ol:** Propargyl alcohol (CHCCH₂OH) (7.4 g, 132 mmol) was fed into ethylmagnesium chloride (C₂H₅MgCl) (2 M) (120 mL) at room temperature over 1 hour while the temperature rose to 50 °C. After the feed was complete, the reaction was heated to reflux for 1 hour and then cooled to -78 °C. The mixture of 1,1-dimethyl-octahydro-inden-5-one and 3,3-dimethyl-octahydro-inden-5-one (obtained as above in EXAMPLE VIII) (16 g, 96 mmol) was fed in dropwise over 15 minutes. The reaction was subsequently allowed to warm to room temperature over 3.5 hours. Saturated aqueous ammonium chloride (NH₄Cl) (50 mL) was added with ethyl acetate (CH₃COOC₂H₅) (50 mL). The resulting organic layer was separated and evaporated to dryness. Silica gel chromatography (ethyl acetate:hexanes = 1:1) provided the mixture of 5-(3-hydroxy-prop-1-ynyl)-1,1-dimethyl-octahydro-inden-5-ol and 5-(3-hydroxy-prop-1-ynyl)-3,3-dimethyl-octahydro-inden-5-ol in a weight ratio of 40:60 (6 g).

The mixture of 5-(3-hydroxy-prop-1-ynyl)-1,1-dimethyl-octahydro-inden-5-ol and 5-(3-hydroxy-prop-1-ynyl)-3,3-dimethyl-octahydro-inden-5-ol was described as having weak and non-descript characters.

The mixture of 5-(3-hydroxy-prop-1-ynyl)-1,1-dimethyl-octahydro-inden-5-ol and 5-(3-hydroxy-prop-1-ynyl)-3,3-dimethyl-octahydro-inden-5-ol possessed the NMR spectral characteristics of:
¹H NMR (CDCl₃, 500 MHz): 4.21-4.27 (m, 2H), 2.20-3.00 (m, 3H), 1.23-1.95 (m, 11H), 0.83-0.99 (m, 6H)

### REFERENCE EXAMPLE X

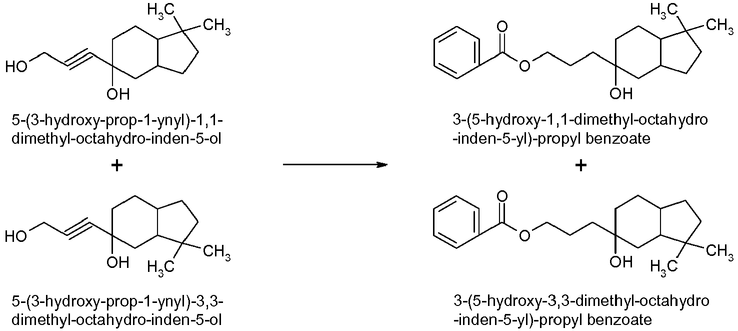

**Preparation of 3-(5-Hydroxy-1,1-dimethyl-octahydro-inden-5-yl)-propyl Benzoate and 3-(5-Hydroxy-3,3-dimethyl-octahydro-inden-5-yl)-propyl Benzoate:** The mixture of 5-(3-hydroxy-prop-1-ynyl)-1,1-dimethyl-octahydro-inden-5-ol and 5-(3-hydroxy-prop-1-ynyl)-3,3-dimethyl-octahydro-inden-5-ol (obtained as above in EXAMPLE IX) (5 g, 22.5 mmol) in isopropanol (25 mL) was hydrogenated over palladium on aluminum oxide (Pd/Al₂O₃) (50 mg) under 100 psi (689.48 kPa) H₂ to the saturated product. The resulting mixture was filtered through Celite® and evaporated to dryness. The crude was dissolved in tetrahydrofuran (THF) (30 mL). To the crude solution were added triethylamine (N(CH₂CH₃)₃) (5 mL) and benzoyl chloride (C₆H₅COCl) (3.5 g, 25 mmol). The reaction mixture was further stirred at room temperature for 10 hours and washed with saturated sodium bicarbonate (NaHCO₃). Silica gel chromatography (ethyl acetate:hexanes = 1:1) provided a pale yellow oil comprising the mixture of 3-(5-hydroxy-1,1-dimethyl-octahydro-inden-5-yl)-propyl benzoate and 3-(5-hydroxy-3,3-dimethyl-octahydro-inden-5-yl)-propyl benzoate in a weight ratio of 40:60 (5.1 g).

The mixture of 3-(5-hydroxy-1,1-dimethyl-octahydro-inden-5-yl)-propyl benzoate and 3-(5-hydroxy-3,3-dimethyl-octahydro-inden-5-yl)-propyl benzoate possessed the NMR spectral characteristics of:
¹H NMR (CDCl₃, 500 MHz): 7.95-8.01 (m, 2H), 7.45-7.52 (m, 1H), 7.34-7.40 (m, 2H), 4.27 (m, 2H), 2.16-2.46 (m, 1H), 1.10-1.90 (m, 16H), 0.75-1.03 (m, 6H)

### REFERENCE EXAMPLE XI

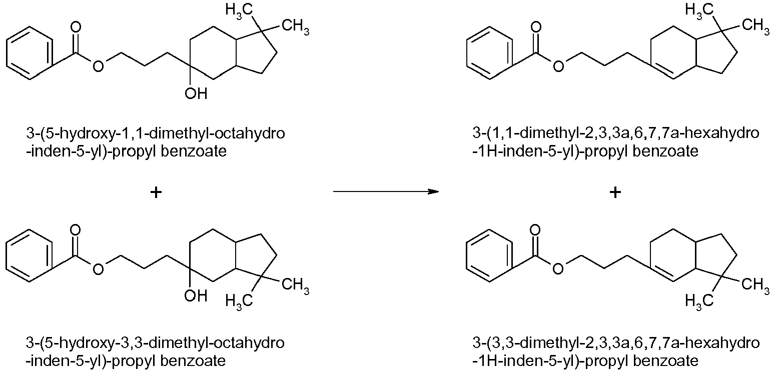

**Preparation of 3-(1,1-Dimethyl-2,3,3a,6,7,7a-hexahydro-1H-inden-5-yl)-propyl Benzoate and 3-(3,3-Dimethyl-2,3,3a,6,7,7a-hexahydro-1H-inden-5-yl)-propyl Benzoate:** The mixture of 3-(5-hydroxy-1,1-dimethyl-octahydro-inden-5-yl)-propyl benzoate and 3-(5-hydroxy-3,3-dimethyl-octahydro-inden-5-yl)-propyl benzoate (obtained as above in EXAMPLE X) (2.4 g, 7.7 mmol) and *para*-toluenesulfonic acid (*p*TSA) (200 mg) in toluene (15 mL) was refluxed for 1 hour. The reaction was cooled and neutralized via washing with saturated aqueous sodium bicarbonate (25 mL). Silica gel chromatography (ethyl acetate:hexanes = 1:1) provided the mixture of 3-(1,1-dimethyl-2,3,3a,6,7,7a-hexahydro-1H-inden-5-yl)-propyl benzoate and 3-(3,3-dimethyl-2,3,3a,6,7,7a-hexahydro-1H-inden-5-yl)-propyl benzoate in a weight ratio of 40:60 (2.3 g).

The mixture of 3-(1,1-dimethyl-2,3,3a,6,7,7a-hexahydro-1H-inden-5-yl)-propyl benzoate and 3-(3,3-dimethyl-2,3,3a,6,7,7a-hexahydro-1H-inden-5-yl)-propyl benzoate possessed the NMR spectral characteristics of:
¹H NMR (CDCl₃, 400 MHz): 7.97-8.17 (m, 2H), 7.52-7.64 (m, 1H), 7.42-7.49 (m, 2H), 5.37-5.57 (m, 1H), 4.26-4.43 (m, 2H), 1.10-2.77 (m, 14H), 0.72-1.10 (m, 6H)

### REFERENCE EXAMPLE XII

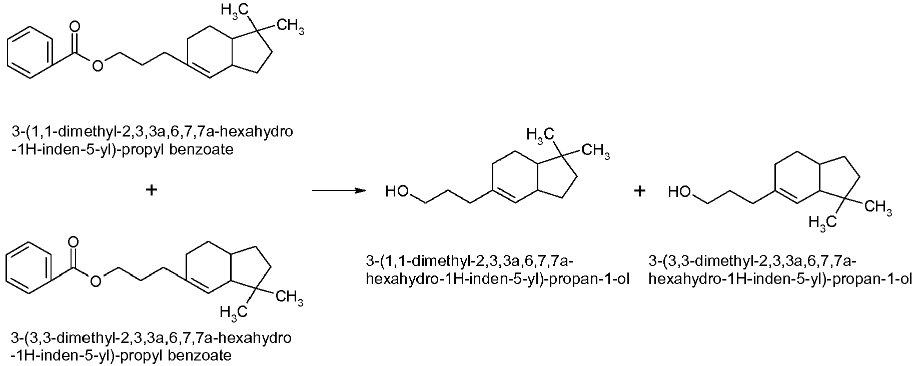

**Preparation of 3-(1,1-Dimethyl-2,3,3a,6,7,7a-hexahydro-1H-inden-5-yl)-propan-1-ol (Formula VIIa) and 3-(3,3-Dimethyl-2,3,3a,6,7,7a-hexahydro-1H-inden-5-yl)-propan-1-ol (Formula VIIb):** The mixture of 3-(1,1-dimethyl-2,3,3a,6,7,7a-hexahydro-1H-inden-5-yl)-propan-1-ol (Formula VIIa) and 3-(3,3-dimethyl-2,3,3a,6,7,7a-hexahydro-1H-inden-5-yl)-propan-1-ol (Formula VIIb) in a weight ratio of 40:60 (2.6 g) was prepared similarly according to EXAMPLE VII using the mixture of 3-(1,1-dimethyl-2,3,3a,6,7,7a-hexahydro-1H-inden-5-yl)-propyl benzoate and 3-(3,3-dimethyl-2,3,3a,6,7,7a-hexahydro-1H-inden-5-yl)-propyl benzoate (obtained as above in EXAMPLE XI) (4.0 g, 13 mmol).

The mixture of Formula VIIa and VIIb was described as having weak floral and muguet notes with dirty character. Appeared gasoline-like and very harsh.

The mixture of Formula VIIa and VIIb possessed the NMR spectral characteristics of:
¹H NMR (CDCl₃, 500 MHz): 5.35-5.56 (m, 1H), 3.66 (t, J=6.5 Hz, 2H), 1.21-2.95 (m, 15H), 0.76-1.09 (m, 6H)

### REFERENCE EXAMPLE XIII

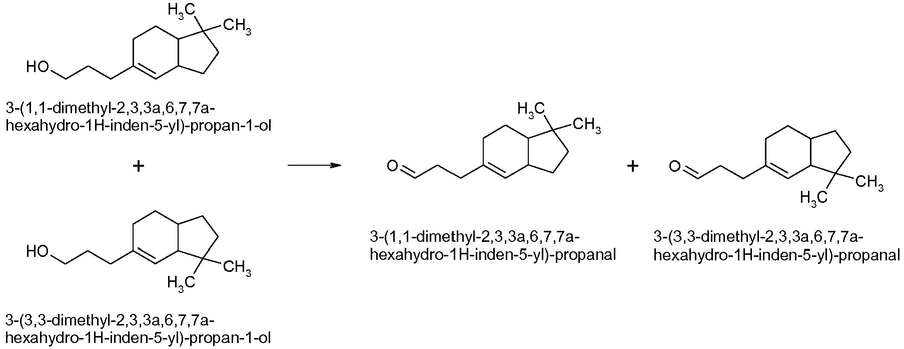

### Preparation of 3-(1,1-Dimethyl-2,3,3a,6,7,7a-hexahydro-1H-inden-5-yl)-propanal (Formula VIIIa) and 3-(3,3-Dimethyl-2,3,3a,6,7,7a-hexahydro-1H-inden-5-yl)-propanal (Formula VIIIb):

The mixture of 3-(1,1-dimethyl-2,3,3a,6,7,7a-hexahydro-1H-inden-5-yl)-propan-1-ol and 3-(3,3-dimethyl-2,3,3a,6,7,7a-hexahydro-1H-inden-5-yl)-propan-1-ol (obtained as above in EXAMPLE XII) (1 g, 4.8 mmol) was dissolved in dichloromethane (100 mL) and treated with pyridinium chlorochromate (C₅H₅NH[CrO₃Cl]) (1.5 g, 7.2 mmol). The reaction mixture was stirred at room temperature for 2 hours and poured into ether (200 mL). The resulting mixture was filtered through Celite® and evaporated to dryness. Silica gel chromatography (ethyl acetate:hexanes = 1:100) provided the mixture of 3-(1,1-dimethyl-2,3,3a,6,7,7a-hexahydro-1H-inden-5-yl)-propanal (Formula VIIIa) and 3-(3,3-dimethyl-2,3,3a,6,7,7a-hexahydro-1H-inden-5-yl)-propanal (Formula VIIIb) in a weight ratio of 30:60 (0.3 g).

The mixture of Formula VIIIa and VIIIb was described as having floral and muguet notes with solventy and sour characters. Appeared weak and simple.

3-(1,1-Dimethyl-2,3,3a,6,7,7a-hexahydro-1H-inden-5-yl)-propanal:
¹H NMR (CDCl₃, 500 MHz): 9.73 (t, J=2.2Hz, 1H), 5.42-5.46 (m, 1H), 1.15-2.72 (m, 14H), 0.97 (s, 3H), 0.95 (s, 3H)

3-(3,3-Dimethyl-2,3,3a,6,7,7a-hexahydro-1H-inden-5-yl)-propanal:
¹H NMR (CDCl₃, 500 MHz): 9.74 (t, J=2.2Hz, 1H), 5.32-5.40 (m, 1H), 1.15-2.72 (m, 14H), 1.00 (s, 3H), 0.79 (s, 3H)

### REFERENCE EXAMPLE XIV

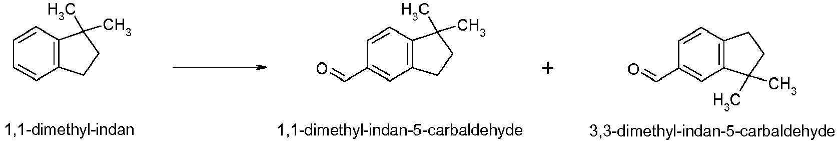

### Preparation of 1,1-Dimethyl-indan-5-carbaldehyde and 3,3-Dimethyl-indan-5-carbaldehyde:

1,1-Dimethyl-indan (641 g, 4.4 mol) was treated with 1,3,5,7-tetra-azaadamantae ((CH₂)₆N₄) (615 g, 4.4 mol) in trifluoroacetic acid (CF₃CO₂H) (5 L), heated to 80 °C for 8 hours and cooled to room temperature. Water (1 L) and toluene (1 L) were added and the reaction mixture was stirred for 3 hours. The reaction mixture was subsequently heated to 130 °C to remove the solvent and cooled again to room temperature. Water (2 L) and toluene (1 L) were added. The organic layer was separated. Further short path distillation provided the mixture of 1,1-dimethyl-indan-5-carbaldehyde and 3,3-dimethyl-indan-5-carbaldehyde in a weight ratio of 1:1 (120 g).

1,1-Dimethyl-indan-5-carbaldehyde:
¹H NMR (CDCl₃, 500 MHz): 9.92 (s, 1H), 7.59-7.71 (m, 2H), 7.22-7.31 (m, 1H), 2.89-2.95 (m, 2H), 1.92-1.98 (m, 2H), 1.26 (s, 6H)

3,3-Dimethyl-indan-5-carbaldehyde:
¹H NMR (CDCl₃, 500 MHz): 9.94 (s, 1H), 7.59-7.71 (m, 2H), 7.22-7.31 (m, 1H), 2.89-2.95 (m, 2H), 1.92-1.98 (m, 2H), 1.27 (s, 6H)

### REFERENCE EXAMPLE XV

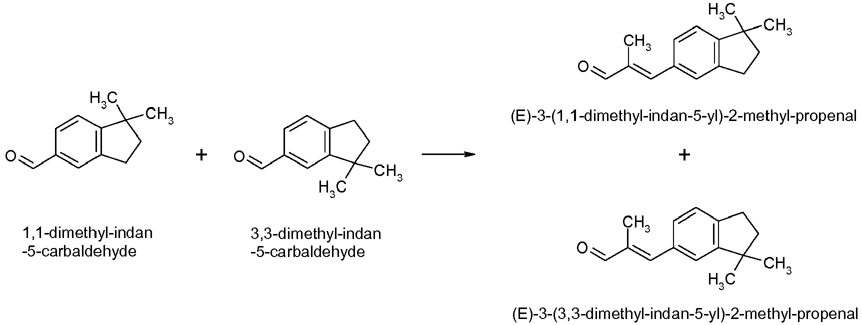

**Preparation of (E)-3-(1,1-Dimethyl-indan-5-yl)-2-methyl-propenal (Formula IXa) and (E)-3-(3,3-Dimethyl-indan-5-yl)-2-methyl-propenal (Formula IXb):** The mixture of 1,1-dimethyl-indan-5-carbaldehyde and 3,3-dimethyl-indan-5-carbaldehyde (obtained as above in EXAMPLE XIV) (171 g, 0.98 mole) was treated with potassium hydroxide (5.51 g, 98 mmol) in methanol (400 mL). Propionaldehyde (C₂H₅CHO) (80 g, 1.4 mol) was fed over 4 hours while the temperature was maintained between 25-35 °C. After the feed was complete, water (1 L), acetic acid (10 mL) and toluene (1 L) were added. The resulting aqueous layer was discarded. The organic layer was placed back in the reaction vessel with *p*-tolouene sulfonic acid (1 g) and heated to reflux for 1 hour to remove water. The reaction was then cooled, washed with water and distilled to provide the mixture of (E)-3-(1,1-dimethyl-indan-5-yl)-2-methyl-propenal (Formula IXa) and (E)-3-(3,3-dimethyl-indan-5-yl)-2-methyl-propenal (Formula IXb) in a weight ratio of 1:1 (52 g).

The mixture of Formula IXa and IXb was described as having strong floral, green and spicy notes with slightly chemical character.

The mixture of Formula IXa and IXb possessed the NMR spectral characteristics of:
¹H NMR (CDCl₃, 400 MHz): 9.57-9.62 (m, 1H), 7.06-7.49 (m, 4H), 2.93-3.00 (m, 2H), 2.12 (s, 3H), 1.95-2.04 (m, 2H), 1.32 (s, 6H)

### REFERENCE EXAMPLE XVI

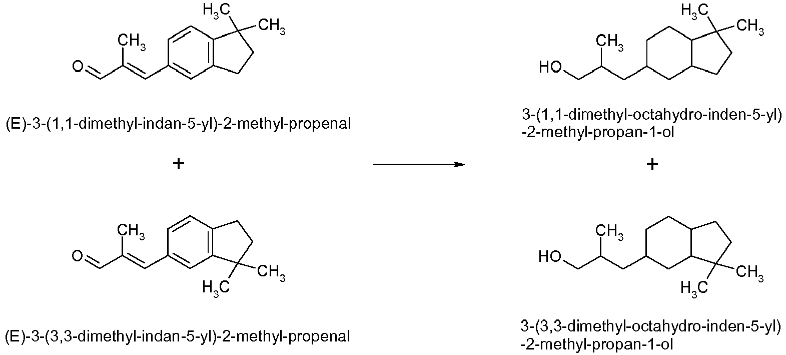

**Preparation of 3-(1,1-Dimethyl-octahydro-inden-5-yl)-2-methyl-propan-1-ol (Formula Xa) and 3-(3,3-Dimethyl-octahydro-inden-5-yl)-2-methyl-propan-1-ol (Formula Xb):** The mixture of 3-(1,1-dimethyl-octahydro-inden-5-yl)-2-methyl-propan-1-ol (Formula Xa) and 3-(3,3-dimethyl-octahydro-inden-5-yl)-2-methyl-propan-1-ol (Formula Xb) in a weight ratio of 1:1 (35 g) was prepared similarly according to EXAMPLE IV using the mixture of (E)-3-(1,1-dimethyl-indan-5-yl)-2-methyl-propenal and (E)-3-(3,3-dimethyl-indan-5-yl)-2-methyl-propenal (obtained as above in EXAMPLE XV) (55 g, 0.22 mol).

The mixture of Formula Xa and Xb was described as having gassy, oily and dirty notes. Floral and fruity notes appeared weak and suppressed.

The mixture of Formula Xa and Xb possessed the NMR spectral characteristics of:
¹H NMR (CDCl₃, 400 MHz): 3.35-3.61 (m, 2H), 0.50-2.50 (m, 26H)

### REFERENCE EXAMPLE XVII

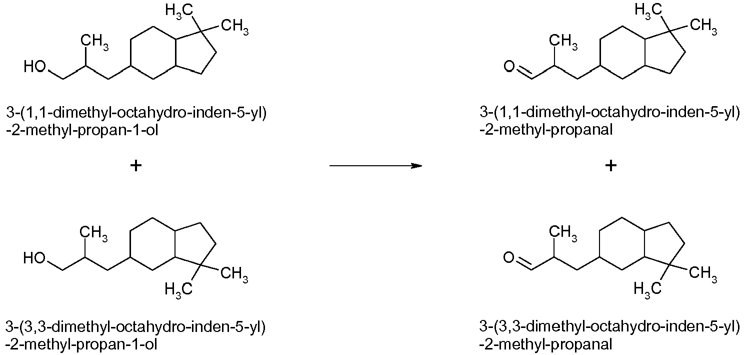

**Preparation of 3-(1,1-Dimethyl-octahydro-inden-5-yl)-2-methyl-propanal (Formula XIa) and 3-(3,3-Dimethyl-octahydro-inden-5-yl)-2-methyl-propanal (Formula XIb):** The mixture of 3-(1,1-dimethyl-octahydro-inden-5-yl)-2-methyl-propanal (XIa) and 3-(3,3-dimethyl-octahydro-inden-5-yl)-2-methyl-propanal (XIb) in a weight ratio of 1:1 (0.3 g) was prepared similarly according to EXAMPLE XIII using the mixture of 3-(1,1-dimethyl-octahydro-inden-5-yl)-2-methyl-propan-1-ol and 3-(3,3-dimethyl-octahydro-inden-5-yl)-2-methyl-propan-1-ol (obtained as above in EXAMPLE XVI) (1.0 g, 4.5 mmol).

The mixture of Formula XIa and XIb was described as having floral, muguet and fatty notes. Appeared weak and simple.

The mixture of Formula XIa and XIb possessed the NMR spectral characteristics of:
¹H NMR (CDCl₃, 400 MHz): 9.49 (s, 1H), 0.60-2.40 (m, 27H)

### REFERENCE EXAMPLE XVIII

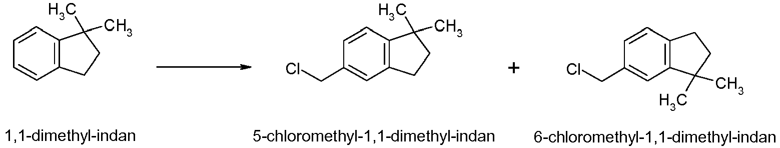

### Preparation of 5-Chloromethyl-1,1-dimethyl-indan and 6-Chloromethyl-1,1-dimethyl-indan:

1,1-Dimethyl-indan (146 g, 1 mol), formaldehyde (HCHO) (37%) (122 g, 1.5 mol) and HCl (12 M, 500 mL) were combined, heated to 60 °C for 8 hours and cooled. The unreacted raw material was removed via fractional distillation to provide a clear oil comprising the mixture of 5-chloromethyl-1,1-dimethyl-indan and 6-chloromethyl-1,1-dimethyl-indan in a weight ratio of 1:1 (60 g).

The mixture of 5-chloromethyl-1,1-dimethyl-indan and 6-chloromethyl-1,1-dimethyl-indan possessed the NMR spectral characteristics of:
¹H NMR (CDCl₃, 400 MHz): 7.18-7.41 (m, 3H), 4.64-4.74 (m, 2H), 2.95-3.05 (m, 2H), 2.04 (m, 2H), 1.38 (s, 6H)

### REFERENCE EXAMPLE XIX

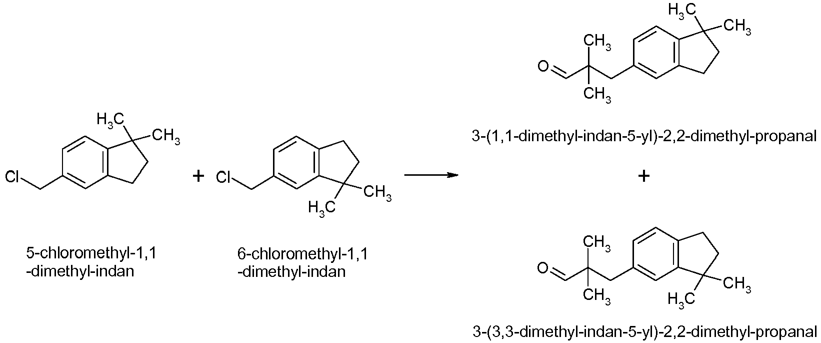

**Preparation** of **3-(1,1-Dimethyl-indan-5-yl)-2,2-dimethyl-propanal (Formula XIIa) and 3-(3,3-Dimethyl-indan-5-yl)-2,2-dimethyl-propanal (Formula XIIb):** Aqueous sodium hydroxide (50%) (17.56 g, 220 mmol), triethylamine (889 mg, 8.8 mmol), sodium iodide (NaI) (1.32 g, 889 mmol) and water (5.5 mL) were combined with toluene (100 mL) and heated to 70 °C. The mixture of 5-chloromethyl-1,1-dimethyl-indan and 6-chloromethyl-1,1-dimethyl-indan (obtained as above in EXAMPLE XVIII) (42 g, 220 mmol) and isobuteraldehyde ((CH₃)₂CHCHO) (15.8 g, 220 mmol) were fed in over 7 hours. The reaction was then aged for 1 hour and subsequently cooled to room temperature. The organic layer was washed with water and fractionally distilled to provide the mixture of 3-(1,1-dimethyl-indan-5-yl)-2,2-dimethyl-propanal (Formula XIIa) and 3-(3,3-dimethyl-indan-5-yl)-2,2-dimethyl-propanal (Formula XIIb) in a weight ratio of 1:1 (40 g).

The mixture of Formula XIIa and XIIb was described as having green and ozonic notes. Appeared weak, simple and chemical.

The mixture of Formula XIIa and XIIb possessed the NMR spectral characteristics of:
¹H NMR (CDCl₃, 500 MHz): 9.63 (s, 1H), 6.83-7.22 (m, 3H), 2.84-3.00 (m, 2H), 2.72-2.84 (m, 2H), 1.87-2.03 (m, 2H), 1.28 (s, 6H), 1.07-1.10 (m, 6H)

### REFERENCE EXAMPLE XX

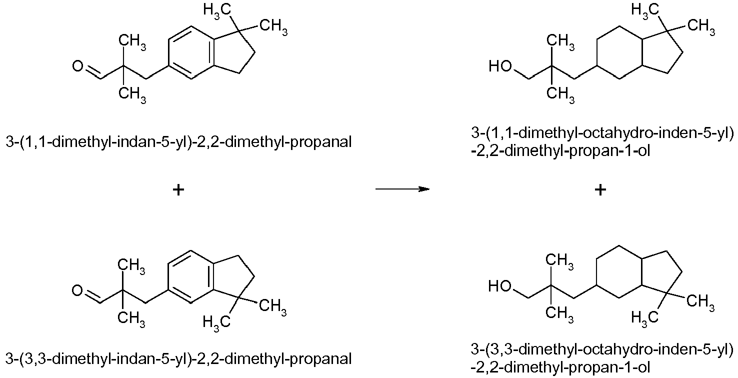

### Preparation of 3-(1,1-Dimethyl-octahydro-inden-5-yl)-2,2-dimethyl-propan-1-ol (Formula XIIIa) and 3-(3,3-Dimethyl-octahydro-inden-5-yl)-2,2-dimethyl-propan-1-ol (Formula XIIIb):

The mixture of 3-(1,1-dimethyl-octahydro-inden-5-yl)-2,2-dimethyl-propan-1-ol (Formula XIIIa) and 3-(3,3-dimethyl-octahydro-inden-5-yl)-2,2-dimethyl-propan-1-ol (Formula XIIIb) in a weight ratio of 1:1 (30 g) was prepared similarly according to EXAMPLE IV using the mixture of 3-(1,1-dimethyl-indan-5-yl)-2,2-dimethyl-propanal and 3-(3,3-dimethyl-indan-5-yl)-2,2-dimethyl-propanal (obtained as above in EXAMPLE XIX) (40 g, 0.18 mol).

The mixture of Formula XIIIa and XIIIb was described as having fruity and strawberry notes but were volatile and not long-lasting. Appeared simple and thin.

The mixture of Formula XIIIa and XIIIb possessed the NMR spectral characteristics of:
¹H NMR (CDCl₃, 500 MHz): 3.27-3.33 (m, 2H), 0.58-2.45 (m, 28H)

### REFERENCE EXAMPLE XXI

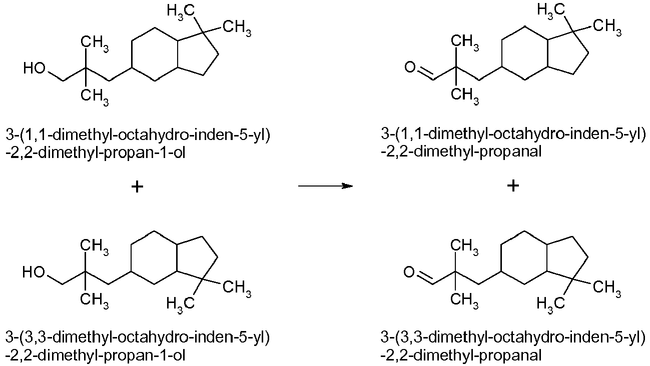

**Preparation of 3-(1,1-Dimethyl-octahydro-inden-5-yl)-2,2-dimethyl-propanal (Formula XIVa) and 3-(3,3-Dimethyl-octahydro-inden-5-yl)-2,2-dimethyl-propanal (Formula XIVb):** The mixture of 3-(1,1-dimethyl-octahydro-inden-5-yl)-2,2-dimethyl-propanal (Formula XIVa) and 3-(3,3-dimethyl-octahydro-inden-5-yl)-2,2-dimethyl-propanal (Formula XIVb) in a weight ratio of 1:1 ( 0.45 g) was prepared similarly according to EXAMPLE XIII using the mixture of 3-(1,1-dimethyl-octahydro-inden-5-yl)-2,2-dimethyl-propan-1-ol and 3-(3,3-dimethyl-octahydro-inden-5-yl)-2,2-dimethyl-propan-1-ol (obtained as above in EXAMPLE XX) (1.0 g, 4.19 mmol).

The mixture of Formula XIVa and XIVb was described as having floral and muguet notes with fatty and sweaty characters.

The mixture of Formula XIVa and XIVb possessed the NMR spectral characteristics of:
¹H NMR (CDCl₃, 400 MHz): 9.49 (s, 1H), 0.60-2.40 (m, 27H)

### REFERENCE EXAMPLE XXII

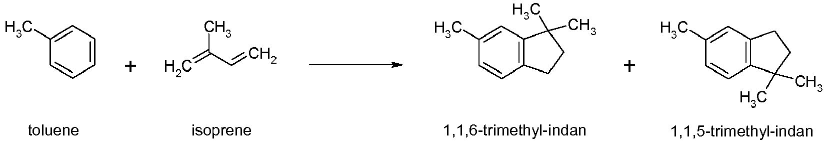

**Preparation of 1,1,6-Trimethyl-indan and 1,1,5-Trimethyl-indan:** Toluene (3.2 Kg, 35 mol) was combined with sulfuric acid (H₂SO₄) (450 g, 4.2 mol) and water (100 mL). The mixture was cooled to 0 °C. Isoprene (823 g, 12.2 mol) was added to the reaction mixture over 6 hours. The reaction mixture was stirred for additional 2 hours and poured into a separatory funnel. The aqueous layer was discarded. The organic layer was washed with aqueous sodium hydroxide (500 mL, 30%). Further distillation provided the mixture of 1,1,6-trimethyl-indan and 1,1,5-trimethyl-indan in a weight ratio of 30:70 (740 g).

¹H NMR (CDCl₃, 400 MHz): 6.76-7.40 (m, 3H), 2.77-2.92 (m, 2H), 2.38 (s, 68% of 3H), 2.29 (s, 32% of 3H), 1.90-2.02 (m, 2H), 1.29 (s, 32% of 6H), 1.28 (s, 68% of 6H)

### REFERENCE EXAMPLE XXIII

**Preparation of 1-(1,1,6-Trimethyl-indan-5-yl)-ethanone and 1-(3,3,6-Trimethyl-indan-5-yl)-ethanone:** The mixture of 1-(1,1,6-trimethyl-indan-5-yl)-ethanone and 1-(3,3,6-trimethyl-indan-5-yl)-ethanone in a weight ratio of 20:80 (337 g) was prepared similarly according to EXAMPLE III using the mixture of 1,1,6-trimethyl-indan and 1,1,5-trimethyl-indan (obtained as above in EXAMPLE XXII) (300 g, 1.87 mol).

The mixture of 1-(1,1,6-trimethyl-indan-5-yl)-ethanone and 1-(3,3,6-trimethyl-indan-5-yl)-ethanone was described as having solventy and kerosene-like characters.

¹H NMR (CDCl₃, 400 MHz): 7.58 (s, 1H), 7.02 (s, 1H), 2.92 (t, J= 7.2 Hz, 79% of 1H), 2.89 (t, J=7.2 Hz, 21% of 1H), 2.58 (s, 3H), 2.56 (s, 79% of 3H), 2.45 (s, 21% of 3H), 1.97 (t, J= 7.1 Hz, 2H), 1.29 (s, 3H)

### EXAMPLE XXIV

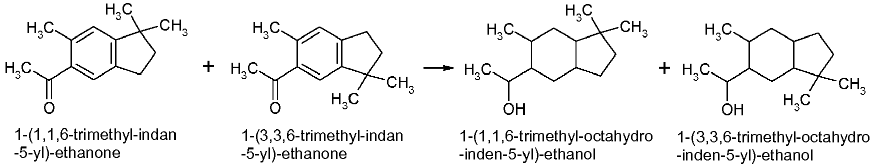

**Preparation of 1-(1,1,6-Trimethyl-octahydro-inden-5-yl)-ethanol (Formula XVa) and 1-(3,3,6-Trimethyl-octahydro-inden-5-yl)-ethanol (Formula XVb):** The mixture of 1-(1,1,6-trimethyl-octahydro-inden-5-yl)-ethanol (Formula XVa) and 1-(3,3,6-trimethyl-octahydro-inden-5-yl)-ethanol (Formula XVb) in a weight ratio of 20:80 (450 g) was prepared similarly according to EXAMPLE IV using the mixture of 1-(1,1,6-trimethyl-indan-5-yl)-ethanone and 1-(3,3,6-trimethyl-indan-5-yl)-ethanone (obtained as above in EXAMPLE XXIII) (505 g, 2.5 mol).

The mixture of Formula XVa and XVb was described as having woody and earthy notes with a particularly cedar-like character.

¹H NMR (CDCl₃, 500 MHz): 3.40-4.17 (m, 1H), 2.16-2.51 (m, 1H), 1.79-2.03 (m, 1H), 1.28-1.79 (m, 8H), 1.08-1.28 (m, 4H), 0.41-1.08 (m, 11H)

### REFERENCE EXAMPLE XXV

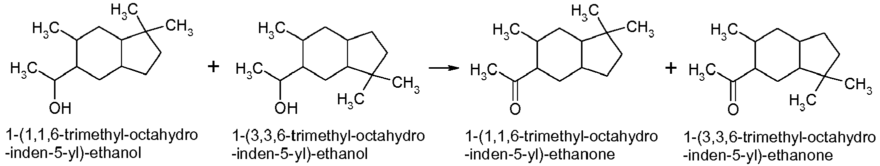

**Preparation of 1-(1,1,6-Trimethyl-octahydro-inden-5-yl)-ethanone (Formula XVIa) and 1-(1,1,6-Trimethyl-octahydro-inden-5-yl)-ethanone (Formula XVIb):** A mixture of 1-(1,1,6-trimethyl-octahydro-inden-5-yl)-ethanol and 1-(3,3,6-trimethyl-octahydro-inden-5-yl)-ethanol (obtained as above in EXAMPLE) above (20 g, 95 mmol) was dissolved in toluene (300 mL) and cooled to 0 °C. To this was added a solution of chromic acid (H₂CrO₄), prepared by combining sodium drichromate (Na₂Cr₂O₇) (17 g, 57 mmol), water (9 mL) and sulfuric acid (1.71 g, 95 mmol). The reaction mixture was stirred at 0 oC for 2 hours and toluene was added (50 mL). The resulting organic layer was separated and evaporated to dryness. Silica gel chromatography (ethyl acetate:hexanes = 9:1) provided the mixture of 1-(1,1,6-trimethyl-octahydro-inden-5-yl)-ethanone (Formula XVIa) and 1-(1,1,6-trimethyl-octahydro-inden-5-yl)-ethanone (Formula XVIb) as a pale oil in a weight ratio of 30:70 (14 g).

The mixture of Formula XVIa and XVIb was described as having herbal, woody, fresh and piney with balsamic character.

¹H NMR (CDCl₃, 400 MHz): 2.42-2.74 (m, 1H), 2.12-2.33 (m, 1H), 2.09 (s, 27% of 3H), 2.07 (s, 73% of 3H), 1.63-2.04 (m, 3H), 1.10-1.62 (m, 7H), 0.69-1.08 (m, 9H)

### EXAMPLE XXVI

The odor profiles of the above compounds were evaluated using (i) odor strength of 0 to 10, where 0 = none, 1 = very weak, 5 = moderate, 10 = extremely strong; and (ii) level of complexity, where 0 = none, 1 = very low, 5 = moderate, 10 = extremely high. The results are listed in the following:

| **Compound** | **Odor Profile** | **Strength** | **Complexity** |
|---|---|---|---|
| Formula Ia/Ib | Floral, muguet, ozonic, orris and waxy; chemical and harsh | 4 | 4 |
| Formula IIa/IIb | Floral, spicy and woody; fruity, citrusy, green and herbaceous; particularly fresh and watery | 8 | 8 |
| Formula IIIa/IIIb | Musky, woody, powdery and sweet | 8 | 9 |
| Formula Va/Vb | Animalic and leathery; clean and herbaceous | 7 | 8 |
| Formula VIa/VIb | Woody, strong leathery and herbaceous | 8 | 8 |
| Formula VIIa/VIIb | Weak floral, muguet and dirty; gasoline-like and harsh | 6 | 2 |
| Formula VIIIa/VIIIb | Floral, muguet, solventy and sour; weak and simple | 3 | 2 |
| Formula Xa/Xb | Gassy, oily and dirty; weak floral and fruity | 3 | 3 |
| Formula XIa/XIb | Floral, muguet and fatty; weak and simple | 2 | 3 |
| Formula XIIa/XIIb | Green and ozonic; weak, simple and chemical | 4 | 3 |
| Formula XIIIa/XIIIb | Fruity and strawberry-like; volatile and not long-lasting; simple and thin | 6 | 4 |
| Formula XVa/XVb | Woody, earthy and cedar-like | 7 | 9 |
| Formula XVIa/XVIb | Herbal, woody, fresh, piney and balsamic | 7 | 7 |
| 3-(1,1-Dimethyl-indan-5-yl)-propanal and 3-(3,3-dimethyl-indan-5-yl)-propanal | Aldehydic, muguet, floral, fresh and aggressive | 9 | 6 |

The above evaluation demonstrated that the mixtures of Formula IIa and IIb, Formula IIIa and IIIb, Formula Va and Vb, Formula VIa and VIb and Formula XVa and XVb displayed highly desirable properties with no off-notes. Thus, these compounds are superior to their close analogs. The advantageous and distinctive properties of these mixtures are unexpected and would not have been predicted. Compounds represented by Formulas Ia, Ib, Iva, IVb, VIIa, VIIb, VIIIa, VIIIb, IXa, IXb, Xa, Xb, XIa, XIb, XIIa, XIIb, XIIIa, XIIIb, XIVa, XIVb, XVIa and XVIb are for reference only.

### EXAMPLE XXVII

It was further found unexpectedly that the above compounds are particularly suitable to be used in combination with additional fragrance ingredients. Such combinations are exemplified in the following:

| **Compound** | **Odor Profile** |
|---|---|
| Formula IIa/IIb | When used in combination with an additional fragrance compound such as 3(*or* 4)-(4-hydroxy-4-methylpentyl)-3-cyclohexene-1-carboxaldehyde, 3-(4-*tert-*butylphenyl)-2-methylpropanal, methyl 2-(3-oxo-2-pentylcyclopentyl)acetate, 3-methyl-4-phenyl-2-butanol, 4-isobutyl cyclohexanepropanal, 1-ethoxy-4-(tert-pentyl)cyclohexane or 2-isobutyl-4-methyltetrahydro-2H-pyran-4-ol, Formula IIa/IIb provided very pleasant and desirable watery character, added substantiality and increased the freshness of floralcy without any interference or intrusion. Thus, Formula IIa/IIb offers the advantageous use at a range of concentrations in such combinations. |
| Formula IIIa/IIIb | When used in combination with a musky ingredient such as 3-methylcyclopentadecanone, 3-methylcyclopentadec-5-en-1-one, 3-methylcyclopentadec-2-en-1-one or 4,6,6,7,8,8-hexamethyl-1,3,4,6,7,8-hexahydrocyclopenta[*g*]isochromene, Formula IIIa/IIIb strengthened the odors of the combination, while provided soft and powdery character which represents "feminine" aspect that is appealing and desirable. The combinations exhibited more dimensions and higher complexity. |
| Formula Va/Vb | When used in combination with an additional fragrance compound such as methyl cedryl ketone or 1,2,3,3a,4,5,6,8a-octahydro-2-isopropylidene-4,8-dimethyl-6-azulenol acetate, the leathery note of Formula Va/Vb became cleaner, less animalic, less artificial and more genuine while the woodiness appeared enhanced. |
| Formula VIa/VIb | When used in combination with a certain woody ingredient such as 1-(1,2,3,4,5,6,7,8-octahydro-2,3,8,8-tetramethyl-2-naphthalenyl)ethanone, 1-[(1*R*, 2*R*,8a*S*)-1,2,3,5,6,7,8,8a-octahydro-1,2,8,8-tetramethyl-2-naphthalenyl]-ethanone, 5,6,6-trimethyl-3 -hepten-2 -one, 3,4,5,6,6-pentamethyl-4-hepten-2-one, 3,5,6,6-tetramethyl-4-methylene-2-heptanone or cedarwood oil, the leathery note was cleaner and more pleasant and the woody note was enhanced. The fragrance properties of each ingredient in the combination became more diffusive and noticeable. |
| Formula XVa/XVb | When used in combination with a certain floral ingredient such as 4-isopropylcyclohexylmethanol, methyl 2-(3-oxo-2-pentylcyclopentyl)acetate or 3-methyl-4-phenyl-2-butanol, the woodiness was enhanced and the floralcy remained clear. Formula XVa/XVb provided creaminess and balsamic quality. The combination exhibited warmth, sensuality and beauty. |

As shown, the ingredients in the above combinations were compatible and the combinations possessed particularly desirable, strong and complex odors. Such advantageous properties are unexpected.

## Claims

1. A compound selected from the group consisting of:
1-(1,1-dimethyl-octahydro-inden-5-yl)-ethanol;
1-(3,3-dimethyl-octahydro-inden-5-yl)-ethanol;
1-(1,1-dimethyl-octahydro-inden-5-yl)-ethanone;
1-(3,3-dimethyl-octahydro-inden-5-yl)-ethanone;
1,1-dimethyl-octahydro-inden-5-ol;
3,3 -dimethyl-octahydro-inden-5 -ol;
1,1-dimethyl-octahydro-inden-5-one;
3,3 -dimethyl-octahydro-inden-5 -one;
1-(1,1,6-trimethyl-octahydro-inden-5-yl)-ethanol;
1-(3,3,6-trimethyl-octahydro-inden-5-yl)-ethanol; and
a mixture thereof.

2. A fragrance formulation containing an olfactory acceptable amount of a compound selected from the group consisting of:
1-(1,1-dimethyl-octahydro-inden-5-yl)-ethanol;
1-(3,3-dimethyl-octahydro-inden-5-yl)-ethanol;
1-(1,1-dimethyl-octahydro-inden-5-yl)-ethanone;
1-(3,3-dimethyl-octahydro-inden-5-yl)-ethanone;
1,1-dimethyl-octahydro-inden-5-ol;
3,3 -dimethyl-octahydro-inden-5 -ol;
1,1-dimethyl-octahydro-inden-5-one;
3,3 -dimethyl-octahydro-inden-5 -one;
1-(1,1,6-trimethyl-octahydro-inden-5-yl)-ethanol;
1-(3,3,6-trimethyl-octahydro-inden-5-yl)-ethanol; and
a mixture thereof.

3. The fragrance formulation of claim 2, wherein the olfactory acceptable amount is from 0.005 to 50 weight percent of the fragrance formulation.

4. The fragrance formulation of claim 2, wherein the olfactory acceptable amount is from 0.5 to 25 weight percent of the fragrance formulation.

5. The fragrance formulation of claim 2, wherein the olfactory acceptable amount is from 1 to 10 weight percent of the fragrance formulation.

6. The fragrance formulation of claim 2 further comprising a material selected from the group consisting of a polymer, an oligomer and a non-polymer.

7. The fragrance formulation of claim 6, wherein the non-polymer is selected from the group consisting of a surfactant, an emulsifier, a fat, a wax, a phospholipid, an organic oil, a mineral oil, a petrolatum, a natural oil, a perfume fixative, a fiber, a starch, a sugar and a solid surface material.

8. The fragrance formulation of claim 7, wherein the solid surface material is selected from the group consisting of zeolite and silica.

9. A method of improving, enhancing or modifying a fragrance formulation through the addition of an olfactory acceptable amount of a compound selected from the group consisting of:
1-(1,1-dimethyl-octahydro-inden-5-yl)-ethanol;
1-(3,3-dimethyl-octahydro-inden-5-yl)-ethanol;
1-(1,1-dimethyl-octahydro-inden-5-yl)-ethanone;
1-(3,3-dimethyl-octahydro-inden-5-yl)-ethanone;
1,1-dimethyl-octahydro-inden-5-ol;
3,3 -dimethyl-octahydro-inden-5 -ol;
1,1-dimethyl-octahydro-inden-5-one;
3,3 -dimethyl-octahydro-inden-5 -one;
1-(1,1,6-trimethyl-octahydro-inden-5-yl)-ethanol;
1-(3,3,6-trimethyl-octahydro-inden-5-yl)-ethanol; and a mixture thereof.

10. The method of claim 9, wherein the olfactory acceptable amount is from 0.005 to 50 weight percent of the fragrance formulation.

11. The method of claim 9, wherein the olfactory acceptable amount is from 0.5 to 25 weight percent of the fragrance formulation.

12. The method of claim 9, wherein the olfactory acceptable amount is from 1 to 10 weight percent of the fragrance formulation.

13. A fragrance product containing an olfactory acceptable amount of the compound of claim 1.

14. The fragrance product of claim 13, wherein the fragrance product is selected from the group consisting of a perfume, a cologne, toilet water, a cosmetic product, a personal care product, a fabric care product, a cleaning product, an air freshener, a bar soap, a liquid soap, a shower gel, a foam bath, a skin care product, a hair care product, a deodorant, an antiperspirant, a feminine care product, a baby care product, a family care product, an air care product, a fragrance delivery system, a disinfectant, a washing agent, a dental and oral hygiene product, a health care and nutritional product and a food product.

15. The fragrance product of claim 14, wherein the cleaning product is selected from the group consisting of a detergent, a dishwashing material, a scrubbing composition, a glass cleaner, a metal cleaner, a countertop cleaner, a floor cleaner, a carpet cleaner, a toilet cleaner and a bleach additive.

16. A method of counteracting malodor in an air space or a substrate comprising the step of introducing a malodor counteracting effective amount of the compound of claim 1.

## Patentansprüche

1. Verbindung, ausgewählt aus der Gruppe bestehend aus:
1-(1,1-Dimethyloctahydroinden-5-yl)ethanol;
1-(3,3-Dimethyloctahydroinden-5-yl)ethanol;
1-(1,1-Dimethyloctahydroinden-5-yl)ethanon;
1-(3,3-Dimethyloctahydroinden-5-yl)ethanon;
1,1-Dimethyloctahydroinden-5-ol;
3,3-Dimethyloctahydroinden-5-ol;
1,1-Dimethyloctahydroinden-5-on;
3,3-Dimethyloctahydroinden-5-on;
1-(1,1,6-Trimethyloctahydroinden-5-yl)ethanol;
1-(3,3,6-Trimethyloctahydroinden-5-yl)ethanol und
einer Mischung davon.

2. Duftstoffformulierung, enthaltend eine olfaktorisch annehmbare Menge einer Verbindung, ausgewählt aus der Gruppe bestehend aus:
1-(1,1-Dimethyloctahydroinden-5-yl)ethanol;
1-(3,3-Dimethyloctahydroinden-5-yl)ethanol;
1-(1,1-Dimethyloctahydroinden-5-yl)ethanon;
1-(3,3-Dimethyloctahydroinden-5-yl)ethanon;
1,1-Dimethyloctahydroinden-5-ol;
3,3-Dimethyloctahydroinden-5-ol;
1,1-Dimethyloctahydroinden-5-on;
3,3-Dimethyloctahydroinden-5-on;
1-(1,1,6-Trimethyloctahydroinden-5-yl)ethanol;
1-(3,3,6-Trimethyloctahydroinden-5-yl)ethanol und
einer Mischung davon.

3. Duftstoffformulierung nach Anspruch 2, wobei die olfaktorisch annehmbare Menge 0,005 bis 50 Gewichtsprozent der Duftstoffformulierung beträgt.

4. Duftstoffformulierung nach Anspruch 2, wobei die olfaktorisch annehmbare Menge 0,5 bis 25 Gewichtsprozent der Duftstoffformulierung beträgt.

5. Duftstoffformulierung nach Anspruch 2, wobei die olfaktorisch annehmbare Menge 1 bis 10 Gewichtsprozent der Duftstoffformulierung beträgt.

6. Duftstoffformulierung nach Anspruch 2, ferner umfassend ein Material aus der Gruppe bestehend aus einem Polymer, einem Oligomer und einem Nichtpolymer.

7. Duftstoffformulierung nach Anspruch 6, wobei das Nichtpolymer aus der Gruppe bestehend aus einem Tensid, einem Emulgator, einem Fett, einem Wachs, einem Phospholipid, einem organischen Öl, einem Mineralöl, einem Petrolatum, einem natürlichen Öl, einem Parfüm-Fixateur, einer Faser, einer Stärke, einem Zucker und einem Material mit fester Oberfläche ausgewählt ist.

8. Duftstoffformulierung nach Anspruch 7, wobei das Material mit fester Oberfläche aus der Gruppe bestehend aus Zeolith und Siliciumdioxid ausgewählt ist.

9. Verfahren zur Verbesserung, Verstärkung oder Modifizierung einer Duftstoffformulierung durch Zugabe einer olfaktorisch annehmbaren Menge einer Verbindung, ausgewählt aus der Gruppe bestehend aus:
1-(1,1-Dimethyloctahydroinden-5-yl)ethanol;
1-(3,3-Dimethyloctahydroinden-5-yl)ethanol;
1-(1,1-Dimethyloctahydroinden-5-yl)ethanon;
1-(3,3-Dimethyloctahydroinden-5-yl)ethanon;
1,1-Dimethyloctahydroinden-5-ol;
3,3-Dimethyloctahydroinden-5-ol;
1,1-Dimethyloctahydroinden-5-on;
3,3-Dimethyloctahydroinden-5-on;
1-(1,1,6-Trimethyloctahydroinden-5-yl)ethanol;
1-(3,3,6-Trimethyloctahydroinden-5-yl)ethanol und
einer Mischung davon.

10. Verfahren nach Anspruch 9, bei dem die olfaktorisch annehmbare Menge 0,005 bis 50 Gewichtsprozent der Duftstoffformulierung beträgt.

11. Verfahren nach Anspruch 9, bei dem die olfaktorisch annehmbare Menge 0,5 bis 25 Gewichtsprozent der Duftstoffformulierung beträgt.

12. Verfahren nach Anspruch 9, bei dem die olfaktorisch annehmbare Menge 1 bis 10 Gewichtsprozent der Duftstoffformulierung beträgt.

13. Duftstoffprodukt, enthaltend eine olfaktorisch annehmbare Menge der Verbindung nach Anspruch 1.

14. Duftstoffprodukt nach Anspruch 13, wobei das Duftstoffprodukt aus einem Parfüm, einem Eau de Cologne, einem Toilettenwasser, einem Kosmetikprodukt, einem Körperpflegeprodukt, einem Textilpflegeprodukt, einem Reinigungsprodukt, einem Lufterfrischer, einer Stückseife, einer Flüssigseife, einem Duschgel, einem Schaumbad, einem Hautpflegeprodukt, einem Haarpflegeprodukt, einem Deodorant, einem Antiperspirant, einem Damenhygieneprodukt, einem Säuglingspflegeprodukt, einem Familienpflegeprodukt, einem Luftpflegeprodukt, einem Duftstoffabgabesystem, einem Desinfektionsmittel, einem Waschmittel, einem Zahn- und Mundhygieneprodukt, einem Gesundheits- und Ernährungsprodukt und einem Lebensmittelprodukt ausgewählt ist.

15. Duftstoffprodukt nach Anspruch 14, wobei das Reinigungsprodukt aus der Gruppe bestehend aus einem Reinigungsmittel, einem Geschirrspülmittel, einem Scheuermittel, einem Glasreiniger, einem Metallreiniger, einem Arbeitsplattenreiniger, einem Bodenreiniger, einem Teppichreiniger, einem Toilettenreiniger und einem Bleichadditiv ausgewählt ist.

16. Verfahren zum Entgegenwirken von schlechtem Geruch in einem Luftraum oder einem Substrat, umfassend den Schritt des Eintragens einer zum Entgegenwirken von schlechtem Geruch wirksamen Menge der Verbindung nach Anspruch 1.

## Revendications

1. Composé choisi dans le groupe constitué par :
1-(1,1-diméthyl-octahydro-indén-5-yl)-éthanol ;
1-(3,3-diméthyl-octahydro-indén-5-yl)-éthanol ;
1-(1,1-diméthyl-octahydro-indén-5-yl)-éthanone ;
1-(3,3-diméthyl-octahydro-indén-5-yl)-éthanone ;
1,1-diméthyl-octahydro-indén-5-ol ;
3,3-diméthyl-octahydro-indén-5-ol ;
1,1-diméthyl-octahydro-indén-5-one ;
3,3-diméthyl-octahydro-indén-5-one ;
1-(1,1,6-triméthyl-octahydro-indén-5-yl)-éthanol ;
1-(3,3,6-triméthyl-octahydro-indén-5-yl)-éthanol ;
et un mélange correspondant.

2. Formulation de fragrance contenant une quantité acceptable sur le plan olfactif d'un composé choisi dans le groupe constitué par :
1-(1,1-diméthyl-octahydro-indén-5-yl)-éthanol ;
1-(3,3-diméthyl-octahydro-indén-5-yl)-éthanol ;
1-(1,1-diméthyl-octahydro-indén-5-yl)-éthanone ;
1-(3,3-diméthyl-octahydro-indén-5-yl)-éthanone ;
1,1-diméthyl-octahydro-indén-5-ol ;
3,3-diméthyl-octahydro-indén-5-ol ;
1,1-diméthyl-octahydro-indén-5-one ;
3,3-diméthyl-octahydro-indén-5-one ;
1-(1,1,6-triméthyl-octahydro-indén-5-yl)-éthanol ;
1-(3,3,6-triméthyl-octahydro-indén-5-yl)-éthanol ;
et un mélange correspondant.

3. Formulation de fragrance selon la revendication 2, la quantité acceptable sur le plan olfactif étant de 0,005 à 50 pour cent en poids de la formulation de fragrance.

4. Formulation de fragrance selon la revendication 2, la quantité acceptable sur le plan olfactif étant de 0,5 à 25 pour cent en poids de la formulation de fragrance.

5. Formulation de fragrance selon la revendication 2, la quantité acceptable sur le plan olfactif étant de 1 à 10 pour cent en poids de la formulation de fragrance.

6. Formulation de fragrance selon la revendication 2 comprenant en outre un matériau choisi dans le groupe constitué par un polymère, un oligomère et un non-polymère.

7. Formulation de fragrance selon la revendication 6, le non-polymère étant choisi dans le groupe constitué par un tensioactif, un émulsifiant, une graisse, une cire, un phospholipide, une huile organique, une huile minérale, un pétrolatum, une huile naturelle, un fixateur de parfum, une fibre, un amidon, un sucre et un matériau à surface solide.

8. Formulation de fragrance selon la revendication 7, le matériau à surface solide étant choisi dans le groupe constitué par une zéolithe et une silice.

9. Procédé d'amélioration, d'augmentation ou de modification d'une formulation de fragrance par l'addition d'une quantité acceptable sur le plan olfactif d'un composé choisi dans le groupe constitué par :
1-(1,1-diméthyl-octahydro-indén-5-yl)-éthanol ;
1-(3,3-diméthyl-octahydro-indén-5-yl)-éthanol ;
1-(1,1-diméthyl-octahydro-indén-5-yl)-éthanone ;
1-(3,3-diméthyl-octahydro-indén-5-yl)-éthanone ;
1,1-diméthyl-octahydro-indén-5-ol ;
3,3-diméthyl-octahydro-indén-5-ol ;
1,1-diméthyl-octahydro-indén-5-one ;
3,3-diméthyl-octahydro-indén-5-one ;
1-(1,1,6-triméthyl-octahydro-indén-5-yl)-éthanol ;
1-(3,3,6-triméthyl-octahydro-indén-5-yl)-éthanol ;
et un mélange correspondant.

10. Procédé selon la revendication 9, la quantité acceptable sur le plan olfactif étant de 0,005 à 50 pour cent en poids de la formulation de fragrance.

11. Procédé selon la revendication 9, la quantité acceptable sur le plan olfactif étant de 0,5 à 25 pour cent en poids de la formulation de fragrance.

12. Procédé selon la revendication 9, la quantité acceptable sur le plan olfactif étant de 1 à 10 pour cent en poids de la formulation de fragrance.

13. Produit de fragrance contenant une quantité acceptable sur le plan olfactif du composé selon la revendication 1.

14. Produit de fragrance selon la revendication 13, le produit de fragrance étant choisi dans le groupe constitué par un parfum, une eau de Cologne, une eau de toilette, un produit cosmétique, un produit de soin personnel, un produit d'entretien de textile, un produit de nettoyage, un rafraîchisseur d'air, un pain de savon, un savon liquide, un gel douche, un bain moussant, un produit de soin de la peau, un produit de soin capillaire, un déodorant, un antitranspirant, un produit d'hygiène féminine, un produit de soin pour bébés, un produit de soins familiaux, un produit d'assainissement de l'air, un système de diffusion de fragrance, un désinfectant, un agent de lavage, un produit dentaire et d'hygiène orale, un produit de soin de santé et nutritionnel et un produit alimentaire.

15. Produit de fragrance selon la revendication 14, le produit de nettoyage étant choisi dans le groupe constitué par un détergent, un matériau pour lave-vaisselle, une composition d'épuration, un produit de nettoyage pour le verre, un produit de nettoyage pour le métal, un produit de nettoyage pour plan de travail, un produit de nettoyage pour le sol, un produit de nettoyage de tapis, un produit de nettoyage de toilettes et un additif de blanchiment.

16. Procédé de lutte contre les mauvaises odeurs dans un espace d'air ou un substrat comprenant l'étape d'introduction d'une quantité efficace de lutte contre les mauvaises odeurs du composé selon la revendication 1.
